# EUROPÄISCHE PATENTSCHRIFT

(11) **EP 1 853 596 B1**
(45) Veröffentlichungstag und Bekanntmachung des Hinweises auf die Patenterteilung: **26.01.2011**
(21) Anmeldenummer: 06708235.4
(22) Anmeldetag: 13.02.2006
(51) Int. Cl.: C07D 413/12, A61K 31/538, A61K 31/423, A61P 11/00

(54) **NEUE, LANGWIRKSAME BETAMIMETIKA ZUR BEHANDLUNG VON ATEMWEGSERKRANKUNGEN**
NOVEL, LONG-ACTING BETAMIMETICS FOR TREATING RESPIRATORY DISEASES
NOUVEAUX BETAMIMETIQUES A EFFET PROLONGE POUR TRAITER DES AFFECTIONS DES VOIES RESPIRATOIRES

(30) Priorität: 19.02.2005 DE 102005007654
(43) Veröffentlichungstag der Anmeldung: 14.11.2007
(73) Patentinhaber: Boehringer Ingelheim International GmbH, 55216 Ingelheim am Rhein (DE); Boehringer Ingelheim Pharma GmbH & Co. KG, 55216 Ingelheim (DE)
(72) Erfinder: KONETZKI, Ingo, 88447 Warthausen (DE); BOUYSSOU, Thierry, 88447 Birkenhard (DE); HOENKE, Christoph, 55218 Ingelheim (DE); SCHNAPP, Andreas, 88400 Biberach (DE)
(74) Vertreter: Hammann, Heinz
(86) Internationale Anmeldenummer: PCT/EP2006/050898
(87) Internationale Veröffentlichungsnummer: WO 2006/087315

(56) Entgegenhaltungen:
- WO-A-2004/045618
- WO-A-2005/077361
- WO-A-2005/111004

## Beschreibung

Die vorliegende Erfindung betrifft Verbindungen der Formel **1,** 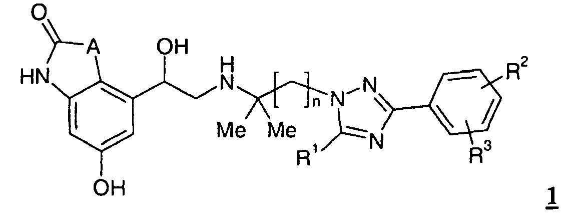 worin n, A, R¹, R² und R³ die in der Beschreibung und in den Ansprüchen genannten Bedeutungen haben können, Verfahren zu deren Herstellung sowie deren Verwendung als Arzneimittel, insbesondere als Arzneimittel zur Behandlung von Atemwegserkrankungen.

### Hintergrund der Erfindung

Betamimetika (β-adrenerge Substanzen) sind aus dem Stand der Technik bekannt. Beispielsweise sei diesbezüglich auf die Offenbarung der WO 04/045618, der WO 01/83462 aber auch älterer Veröffentlichungen, wie beispielsweise der US 4,460,581 oder auch der US 4,154,829 verwiesen. Diese schlagen Betamimetika zur Therapie einer Vielzahl von Erkrankungen vor.

Grundsätzlich ist es bei der medikamentösen Therapie einer Reihe von Erkrankungen häufig wünschenswert, Arzneimittel mit einer längeren Wirkungsdauer bereitzustellen. Hierdurch kann in der Regel gewährleistet werden, dass die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im Übrigen in hohem Maße zum Wohlbefinden des Patienten bei.

Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, dass der Patient sich relativ schnell an die regelmäßige Einnahme des Medikaments zu bestimmten Tageszeiten gewöhnen kann.

Es ist daher Aufgabe der vorliegenden Erfindung, Betamimetika bereitzustellen, die einerseits bei der Therapie von Atemwegserkrankungen einen therapeutischen Nutzen entfalten und darüber hinaus durch eine längere Wirkdauer gekennzeichnet sind und somit zur Herstellung von Arzneimitteln mit längerer Wirksamkeit Verwendung finden können. Es ist insbesondere Aufgabe der vorliegenden Erfindung, Betamimetika bereitzustellen, die aufgrund ihrer langen Wirksamkeit zur Herstellung eines zur Therapie von Atemwegserkrankungen einmal täglich applizierbaren Arzneimittels eingesetzt werden können. Neben den vorstehend genannten Aufgaben ist es ferner Ziel der vorliegenden Erfindung, solche Betamimetika bereitzustellen, die nicht nur außerordentlich potent, sondern ferner durch ein hohes Maß an Selektivität gegenüber dem β₂-Adrenozeptor gekennzeichnet sind.

### Beschreibung der Erfindung

Überraschenderweise wurde gefunden, dass die vorstehend genannten Aufgaben durch Verbindungen der Formel **1** gelöst werden.

Die vorliegende Erfindung betrifft deshalb Verbindungen der Formel **1**, 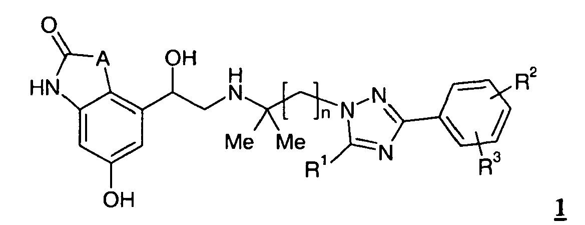 worin
- n: 1, 2 oder 3, bevorzugt 2;
- A: eine zweibindige Gruppe ausgewählt aus der Gruppe bestehend aus -CR⁴R⁵-O-, -CH=CH- oder -CH₂-CH₂-, bevorzugt -CR⁴R⁵-O-;
- R¹: -C₁₋₄-Alkyl;
- R² und R³,: gleich oder verschieden H, C₁₋₄-Alkyl, C₂₋₄-Alkenyl, C₂₋₄-Alkinyl, C₃₋₆-Cycloalkyl, C₁₋₄-Haloalkyl, -O-C₁₋₄-Haloalkyl, Halogen, OH, CN, NO₂ -C₂₋₄-Alkyl-OH, -O-C₁₋₄-Alkyl, COOH oder COO-C₁₋₄-Alkyl, oder R² und R³ gemeinsam eine 2-bindige Gruppe ausgewählt aus -O-CR⁴R⁵-O-, -O-CR⁴R⁵-NR⁶ oder -CH=CH-CH=CH-;
- R⁴: H oder C₁₋₄-Alkyl;
- R⁵: H oder C₁₋₄-Alkyl;
- R⁶: H oder C₁₋₄-Alkyl,
bedeuten, gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

Bevorzugt sind die ferner Verbindungen der Formel **1**, worin
- A: eine zweibindige Gruppe ausgewählt aus der Gruppe bestehend aus -CR⁴R⁵-O-, -CH=CH- oder -CH₂-CH₂-, bevorzugt -CR⁴R⁵-O- mit
- R⁴: H, Methyl, Ethyl, bevorzugt H oder Methyl, besonders bevorzugt H;
- R⁵: H, Methyl, Ethyl, bevorzugt H oder Methyl, besonders bevorzugt H;
bedeuten, und worin n, R¹, R², R³ und R⁶ jeweils eine der vor- oder nachstehend genannten Bedeutungen haben können, gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

Bevorzugt sind die ferner Verbindungen der Formel **1**, worin R¹ Methyl, Ethyl oder Propyl, bevorzugt Methyl oder Ethyl, besonders bevorzugt Methyl bedeutet und worin n, A, R², R³, R⁴, R⁵ und R⁶ jeweils eine der vor- oder nachstehend genannten Bedeutungen haben können, gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

Bevorzugt sind ferner Verbindungen der Formel **1**, worin
- R²: H, Methyl, Ethyl, Propyl, Vinyl, Allyl, Propargyl, Cyclopropyl, Cyclopentyl, Cyclohexyl, -CH₂Cl, -CHCl₂, -CCl₃, -CH₂F, -CHF₂, -CF₃, -CH₂-CH₂Cl, -CH₂-CHCl₂, -CH₂-CCl₃, -CH₂-CH₂F, -CH₂-CHF₂, -CH₂-CF₃, -CH₂-CH₂OH, Fluor, Chlor, Brom, OH, CN, NO₂, Methoxy, Ethoxy, Propoxy, COOH, COO-Methyl, COO-Ethyl, COO-Propyl oder COO-Butyl;
- R³: Methyl, Ethyl, Propyl, Vinyl, Allyl, Propargyl, Cyclopropyl, Cyclopentyl, Cyclohexyl, -CH₂Cl, -CHCl₂, -CCl₃, -CH₂F, -CHF₂, -CF₃, -CH₂-CH₂Cl, -CH₂-CHCl₂, -CH₂-CCl₃, -CH₂-CH₂F, -CH₂-CHF₂, -CH₂-CF₃, -CH₂-CH₂OH, Fluor, Chlor, Brom, OH, CN, NO₂ Methoxy, Ethoxy, Propoxy, COOH, COO-Methyl, COO-Ethyl, COO-Propyl oder COO-Butyl, oder R² und R³ gemeinsam eine 2-bindige Gruppe ausgewählt aus -O-CR⁴R⁵-O-, -O-CR⁴R⁵-NR⁶- oder -CH=CH-CH=CH-;
- R⁴: H, Methyl, Ethyl, bevorzugt H oder Methyl, besonders bevorzugt H;
- R⁵: H, Methyl, Ethyl, bevorzugt H oder Methyl, besonders bevorzugt H;
- R⁶: H, Methyl, Ethyl, bevorzugt H oder Methyl, besonders bevorzugt H;
bedeuten, und worin n, A und R¹ jeweils eine der vor- oder nachstehend genannten Bedeutungen haben können gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

Bevorzugt sind ferner Verbindungen der Formel 1, worin
- R²: H, Methyl, Ethyl, -CF₃, -CH₂-CF₃, Fluor, Chlor, OH, Methoxy, Ethoxy, COOH oder COO-Methyl;
- R³: Methyl, Ethyl, Propyl, Vinyl, Allyl, Cyclopropyl, Cyclopentyl, Cyclohexyl, -CH₂F, -CHF₂, -CF₃, -CH₂-CH₂F, -CH₂-CHF₂, -CH₂-CF₃, -CH₂-CH₂OH, Fluor, Chlor, OH, CN, Methoxy, Ethoxy, COOH, COO-Methyl, COO-Ethyl oder COO-Butyl, oder R²und R³ gemeinsam eine 2-bindige Gruppe ausgewählt aus -O-CH₂-O-, -O-CMe₂-O- oder -CH=CH-CH=CH-; bedeuten, und worin n, A und R¹ jeweils eine der vor- oder nachstehend genannten Bedeutungen haben können gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

Bevorzugt sind ferner Verbindungen der Formel **1**, worin
- R²: H, Methyl, Ethyl, -CF₃, Fluor, Chlor, OH oder Methoxy;
- R³: Methyl, Ethyl, Cyclopropyl, Cyclohexyl, -CF₃, Fluor, Chlor, OH, CN, Methoxy, Ethoxy, COOH, COO-Methyl, COO-Ethyl oder COO-Butyl, oder R² und R³ gemeinsam eine 2-bindige Gruppe ausgewählt aus -O-CH₂-O- oder -CH=CH-CH=CH-;
bedeuten, und worin n, A und R¹ jeweils eine der vor- oder nachstehend genannten Bedeutungen haben können gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

Bevorzugt sind ferner Verbindungen der Formel **1**, worin
- R²: H, Methyl, Fluor, Chlor, OH oder Methoxy;
- R³: Methyl, Ethyl, -CF₃, Fluor, Chlor, OH, Methoxy, Ethoxy, COOH, COO-Methyl oder COO-Butyl, oder R² und R³ gemeinsam eine 2-bindige Gruppe ausgewählt aus -O-CH₂-O- oder -CH=CH-CH=CH-, bevorzugt -O-CH₂-O-;
bedeuten, und worin n, A und R¹ jeweils eine der vor- oder nachstehend genannten Bedeutungen haben können gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

Bevorzugt sind ferner Verbindungen der Formel **1**, worin R² Wasserstoff bedeutet und worin n, A, R¹ und R³ jeweils eine der vor- oder nachstehend genannten Bedeutungen haben können gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

Bevorzugt sind ferner Verbindungen der Formel **1**, worin n 2 bedeutet und worin A, R¹, R² und R³ jeweils eine der vor- oder nachstehend genannten Bedeutungen haben können gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

Bevorzugt sind ferner Verbindungen der Formel **1**, worin
- R³: Methyl, Ethyl, -CF₃, Fluor, Chlor, OH, Methoxy, Ethoxy, COOH, COO-Methyl oder COO-Butyl, bevorzugt Methyl, -CF₃, Fluor, Chlor, OH, Methoxy, COOH oder COO-Methyl, besonders bevorzugt Methyl, -CF₃, Fluor, Chlor, Methoxy oder COOH
bedeuten, und worin n, A, R¹ und R² jeweils eine der vor- oder nachstehend genannten Bedeutungen haben können gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

Ein weiterer Aspekt der vorliegenden Erfindung betrifft die vorstehend genannten neuen Verbindungen der Formel **1** in Form der einzelnen optischen Isomeren, Mischungen der einzelnen Enantiomeren oder Racemate. Besonders bevorzugt sind dabei Verbindungen der Formel **1** in Form der enantiomerenreinen Verbindungen, wobei die R-Enantiomere der

Verbindungen der Formel **1** erfindungsgemäß von herausragender Bedeutung sind. Die R-Enantiomere der Verbindungen der Formel **1** sind darstellbar durch die allgemeine Formel ***R*-1** 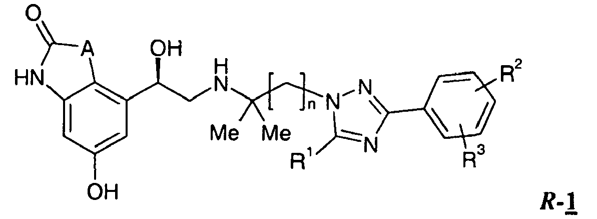 worin n, A, R¹, R² und R³ die vorstehend genannten Bedeutungen haben können.

Verfahren zur Auftrennung von Racematen in die jeweiligen Enantiomere sind im Stand der Technik bekannt und können zur Darstellung der enantiomerenreinen R- bzw. S-Enantiomere der Verbindungen der Formel **1** in analoger Art und Weise zur Anwendung gelangen.

Bevorzugt sind ferner Verbindungen der Formel **1**, worin A -CH₂-O- bedeutet und worin die Reste n, R¹, R² und R³ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate. Von den Verbindungen der Formel **1**, in denen A -CH₂-O- bedeutet, sind diejenigen Regiosiomere bevorzugt, die durch die allgemeine Formel **1.1** gekennzeichnet sind.

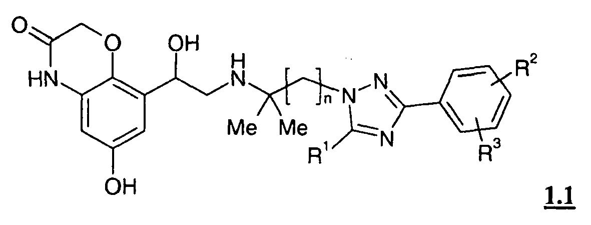

Ein bevorzugter Aspekt der vorliegenden Erfindung betrifft Verbindungen der Formel **1.1** worin n, R¹, R² und R³ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate. Besonders bevorzugt sind erfindungsgemäß die R-Enantiomere der Verbindungen der Formel **1.1**.

Verbindungen der Formel **1**, worin A CH=CH bedeutet sind gekennzeichnet durch die allgemeine Formel **1.2**.

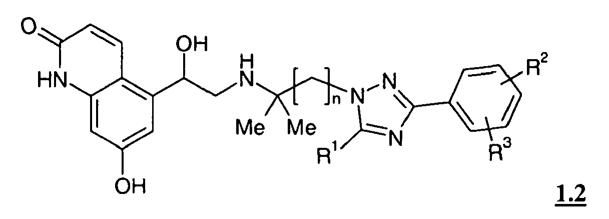

Ein bevorzugter Aspekt der vorliegenden Erfindung betrifft Verbindungen der Formel **1.2** worin n, R¹, R² und R³ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate. Besonders bevorzugt sind erfindungsgemäß die R-Enantiomere der Verbindungen der Formel **1.2**.

Verbindungen der Formel **1**, worin A CH₂-CH₂ bedeutet, sind gekennzeichnet durch die allgemeine Formel **1.3**.

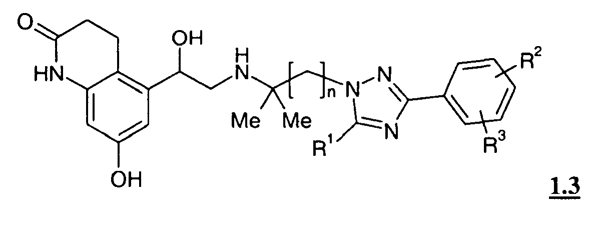

Ein bevorzugter Aspekt der vorliegenden Erfindung betrifft Verbindungen der Formel **1.3** worin n, R¹, R² und R³ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate. Besonders bevorzugt sind erfindungsgemäß die R-Enantiomere der Verbindungen der Formel **1.3.**

Verbindungen der Formel **1**, worin A -CR⁴R⁵-O- und R⁴ bzw. R⁵ Methyl bedeuten, sind ebenfalls erfindungsgemäß bevorzugt. Besonders bevorzugte Regioisomere dieser Strukturklasse sind gekennzeichnet durch die allgemeine Formel **1.4.**

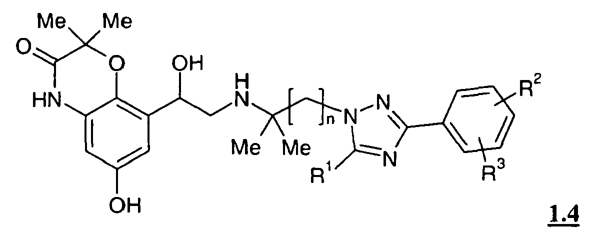

Ein bevorzugter Aspekt der vorliegenden Erfindung betrifft Verbindungen der Formel **1.4** worin n, R¹, R² und R³ die vorstehend genannten Bedeutungen haben können, gegebenenfalls in Form der einzelnen Enantiomere, Mischungen der einzelnen Enantiomere oder Racemate, gegebenenfalls in Form ihrer Säureadditionssalze mit pharmakologisch unbedenklichen Säuren sowie gegebenenfalls in Form der Solvate und/oder Hydrate. Besonders bevorzugt sind erfindungsgemäß die R-Enantiomere der Verbindungen der Formel **1.4**.

Bevorzugt sind ferner Verbindungen der Formel **1**, worin A eine zweibindige Gruppe ausgewählt aus der Gruppe bestehend aus -O-, -CR⁴R⁵- oder -NR⁶- bedeutet und worin n, R¹, R², R³, R⁴, R⁵ und R⁶ jeweils eine der vor- oder nachstehend genannten Bedeutungen haben können gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

Bevorzugt sind ferner Verbindungen der Formel **1**, worin A eine zweibindige Gruppe -S-bedeutet und worin n, R¹, R², und R³ jeweils eine der vor- oder nachstehend genannten Bedeutungen haben können gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

Bevorzugt sind insbesondere Verbindungen der Formel **1**, die ausgewählt sind aus der Gruppe von Verbindungen bestehend aus:
- 8-(2-{3-[3-(4-Fluor-phenyl)-5-methyl-[1,2,4]triazol-1-yl]-1,1-dimethyl-propylamino}-1-hydroxy-ethyl)-6-hydroxy-4H-benzo[1,4]oxazin-3-on;
- 8-{2-[1,1-Dimethyl-3-(5-methyl-3-p-tolyl-[1,2,4]triazol-lyl)-propylamino]-1-hydroxy-ethyl}-6-hydroxy-4H-benzo[1,4]oxazin-3-on;
- 8-(2-{1,1-Dimethyl-3-[5-methyl-3-(4-trifluormethyl-phenyl)-[1,2,4]triazol-1-yl]-propylamino}-1-hydroxy-ethyl)-6-hydroxy-4H-benzo[1,4]oxazin-3-on;
- 8-(2-{3-[3-(3,5-Difluor-phenyl)-5-methyl-[1,2,4]triazol-1-yl]-1,1-dimethyl-propylamino}-1-hydroxy-ethyl)-6-hydroxy-4H-benzo[1,4]oxazin-3-on;
- 3-(1-{3-[2-Hydroxy-2-(6-hydroxy-3-oxo-3,4-dihydro-2H-benzo[1,4]oxazin-8-yl)-ethylamino]-3-methyl-butyl}-5-methyl-1H-[1,2,4]triazol-3-yl-benzoesäure;
- 8-(2-{3-[3-(4-Chlor-phenyl)-5-meihyl-[1,2,4]triazol-1-yl]-1,1-dimethyl-propylamino}-1-hydroxy-ethyl)-6-hydroxy-4H-benzo[1,4]oxazin-3-on;
- 8-(2-{3-[5-Ethyl-3-(4-methoxy-phenyl)-[1,2,4]triazol-1-yl]-1,1-dimethyl-propylamino}-1-hydroxy-ethyl)-6-hydroxy-4H-benzo[1,4]oxazin-3-on;
- 6-Hydroxy-8-(1-hydroxy-2-{3-[3-(4-methoxy-phenyl)-5-methyl-[1,2,4]triazol-1-yl]-1,1-dimethyl-propylaminol-ethyl)-4H-benzo[1,4]oxazin-3-on;
- 8-{2-[3-(3-Benzo[1,3]dioxol-5-yl-5-methyl-[1,2,4]triazol-1-yl)-1,1-dimethyl-propylamino]-1-hydroxy-ethyl}-6-hydroxy-4H-benzo[1,4]oxazin-3-on;
- 7-{2-[3-(3-Benzo[1,3]dioxol-5-yl-5-methyl-[1,2,4]triazol-1-yl)-1,1-dimethyl-propylamino]-1-hydroxy-ethyl}-5-hydroxy-3H-benzooxazol-2-on und
- 8-{2-[3-(3-Benzo[1,3]dioxol-5-yl-5-methyl-[1,2,4]triazol-1-yl)-1,1-dimethyl-propylamino]-1-hydroxy-ethyl}-6-hydroxy-2,2-dimethyl-4H-benzo[1,4]oxazin-3-on, gegebenenfalls in Form ihrer pharmakologisch unbedenklichen Säureadditionssalze, sowie gegebenenfalls in Form ihrer Solvate und/oder Hydrate.

Ein weiterer Aspekt der vorliegenden Erfindung betrifft die vorstehend genannten neuen Verbindungen der Formel **1** als Arzneimittel. Die vorliegende Erfindung betrifft ferner die Verwendung der vorstehend genannten Verbindungen der Formel **1** zur Herstellung eines Arzneimittels zur Behandlung von Atemwegserkrankungen.

Die vorliegende Erfindung betrifft bevorzugt die Verwendung der vorstehend genannten Verbindungen der Formel **1** zur Herstellung eines Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt ist die Verwendung von Verbindungen der Formel **1** zur Herstellung eines Arzneimittels zur Behandlung von Obstruktiven Lungenerkrankungen, die ausgewählt sind aus der Gruppe bestehend aus COPD (chronisch obstruktive pulmonale Erkrankung), Asthma Bronchiale, pädiatrisches Asthma, schweres Asthma, akuter Asthma-Anfall und chronische Bronchitis, wobei die Verwendung zur Herstellung eines Arzneimittels zur Behandlung von Asthma Bronchiale erfindungsgemäß besonders bevorzugt ist.

Bevorzugt ist ferner die Verwendung von Verbindungen der Formel **1** zur Herstellung eines Arzneimittels zur Behandlung von Lungenemphysemen die ihren Ursprung haben in COPD (chronisch obstruktive pulmonale Erkrankung) oder α1-Proteinase-Inhibitor-Mangel.

Bevorzugt ist ferner die Verwendung von Verbindungen der Formel **1** zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

Bevorzugt ist ferner die Verwendung von Verbindungen der Formel **1** zur Herstellung eines Arzneimittels zur Behandlung von Interstitiellen Lungenerkrankungen, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, systemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt ist ferner die Verwendung von Verbindungen der Formel **1** zur Herstellung eines Arzneimittels zur Behandlung von Zystischer Fibrose bzw. Mukoviszidose.

Bevorzugt ist ferner die Verwendung von Verbindungen der Formel **1** zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner die Verwendung von Verbindungen der Formel **1** zur Herstellung eines Arzneimittels zur Behandlung von Bronchiektasen.

Bevorzugt ist ferner die Verwendung von Verbindungen der Formel **1** zur Herstellung eines Arzneimittels zur Behandlung von ARDS (adult respiratory distress syndrom). Bevorzugt ist ferner die Verwendung von Verbindungen der Formel 1 zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt betrifft die vorliegende Erfindung die Verwendung der Verbindungen der Formel **1** zur Herstellung eines Arzneimittels zur Behandlung von Asthma oder COPD. Von besonderer Bedeutung ist ferner die vorstehend genannte Verwendung von Verbindungen der Formel **1** zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Ferner betrifft die vorliegende Erfindung ein Verfahren zur Behandlung der vorstehend genannten Erkrankungen, dadurch gekennzeichnet, daß eine oder mehrere der vorstehend genannten Verbindungen der Formel **1** in therapeutisch wirksamen Mengen appliziert werden. Die vorliegende Erfindung betrifft bevorzugt Verfahren zur Behandlung von Asthma oder COPD, dadurch gekennzeichnet, daß eine oder mehrere der vorstehend genannten Verbindungen der Formel **1** in therapeutisch wirksamen Mengen einmal täglich appliziert werden.

Ein weiterer Aspekt der vorliegenden Erfindung betrifft die vorstehend genannten Verbindungen der Formel **1** in Form ihrer Säureadditionssalze mit pharmakologisch unbedenklichen Säuren sowie gegebenenfalls in Form der Solvate und/oder Hydrate.

Unter Säureadditionssalzen mit pharmakologisch unbedenklichen Säuren werden dabei beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Unter dem Begriff "C₁₋₆-Alkyl" (auch soweit sie Bestandteil anderer Reste sind) werden verzweigte und unverzweigte Alkylgruppen mit 1 bis 6 Kohlenstoffatomen verstanden und unter dem Begriff "C₁₋₄-Alkyl" werden verzweigte und unverzweigte Alkylgruppen mit 1 bis 4 Kohlenstoffatomen verstanden. Bevorzugt sind Alkylgruppen mit 1 bis 4 Kohlenstoffatomen. Beispielsweise werden hierfür genannt: Methyl, Ethyl, *n*-Propyl, *iso-*Propyl, *n*-Butyl, *iso*-Butyl, *sec*-Butyl, *tert*-Butyl, *n*-Pentyl, *iso*-Pentyl, *neo*-Pentyl oder Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₂-₆-Alkenyl (auch soweit sie Bestandteil anderer Reste sind) werden verzweigte und unverzweigte Alkenylgruppen mit 2 bis 6 Kohlenstoffatomen und unter dem Begriff "C₂₋₄-Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatome. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc.

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen. Bevorzugt sind Alkinylgruppen mit 2 bis 4 Kohlenstoffatome. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc.

Unter dem Begriff "C₅₋₆-Cycloalkyl" (auch soweit sie Bestandteil anderer Reste sind) werden cyclische Alkylgruppen mit 5 oder 6 Kohlenstoffatomen verstanden. Beispielsweise werden hierfür genannt: Cyclopentyl oder Cyclohexyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert-*Butyl, Hydroxy, Fluor, Chlor, Brom und Jod.

Unter dem Begriff "C₁₋₆-Haloalkyl" (auch soweit sie Bestandteil anderer Reste sind) werden verzweigte und unverzweigte Alkylgruppen mit 1 bis 6 Kohlenstoffatomen verstanden, die mit einem oder mehreren Halogenatomen substituiert sind. Unter dem Begriff "C₁₋₄-Alkyl" werden verzweigte und unverzweigte Alkylgruppen mit 1 bis 4 Kohlenstoffatomen verstanden, die mit einem oder mehreren Halogenatomen substituiert sind. Bevorzugt sind Alkylgruppen mit 1 bis 4 Kohlenstoffatomen. Bevorzugte Halogenatome sind Fluor, Chlor, besonders bevorzugt Fluor. Beispielsweise werden hierfür genannt: CF₃, CHF₂, CH₂F, CH₂CF₃.

Halogen steht im Rahmen der vorliegenden Erfindung für Fluor, Chlor, Brom oder Jod. Sofern nicht gegenteilig angegeben, gelten Fluor, Chlor und Brom als bevorzugte Halogene.

Verbindungen der allgemeinen Formel **1** können Säuregruppen besitzen, hauptsächlich Carboxylgruppen, und/oder basische Gruppen wie z.B. Aminofunktionen. Verbindungen der allgemeinen Formel **1** können deshalb als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Wie vorstehend genannt, können die Verbindungen der Formel **1** in ihre Salze, insbesondere für die pharmazeutische Anwendung, in ihre physiologisch und pharmakologisch verträglichen Salze überführt werden. Diese Salze können einerseits als physiologisch und pharmakologisch verträgliche Säureadditionssalze der Verbindungen der Formel **1** mit anorganischen oder organischen Säuren vorliegen. Zur Darstellung der Säureadditionssalze kommen beispielsweise Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden. Zur Darstellung der Alkali- und Erdalkalimetallsalze der Verbindung der Formel **1** kommen vorzugsweise die Alkali- und Erdalkalihydroxide und -hydride in Betracht, wobei die Hydroxide und Hydride der Alkalimetalle, besonders des Natriums und Kaliums bevorzugt, Natrium- und Kaliumhydroxid besonders bevorzugt sind.

Gegebenenfalls können die Verbindungen der allgemeinen Formel **1** in ihre Salze, insbesondere für die pharmazeutische Anwendung, in ihre pharmakologisch unbedenklichen Säureadditionssalze mit einer anorganischen oder organischen Säure, überführt werden. Als Säuren kommen hierfür beispielsweise Bernsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure oder Zitronensäure in Betracht. Ferner können Mischungen der vorgenannten Säuren eingesetzt werden.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

Die erfindungsgemäßen Verbindungen können als Racemate vorliegen, sie können aber auch als reine Enantiomere, d.h. in (R)- oder (S)-Form gewonnen werden. Bevorzugt sind Verbindungen die als Racemate bzw. als (R)-Form vorliegen.

Die Herstellung der erfindungsgemäßen Verbindungen kann in Analogie zu im Stand der Technik bereits bekannten Vorgehensweisen erfolgen. Geeignete Herstellverfahren sind beispielsweise aus der US 4,460,581 und 4,154,829 bekannt, auf die an dieser Stelle vollinhaltlich Bezug genommen wird.

Die nachstehenden Beispiele dienen der weitergehenden Veranschaulichung und Verdeutlichung der vorliegenden Erfindung ohne deren Gegenstand allerdings auf die exemplarisch erläuterten Beispiele zu beschränken.

### Synthese der Zwischenprodukte 1-9

### Zwischenprodukt 1: 1,1-Dimethyl-3-(5-methyl-3-p-tolyl-[1,2,4]triazol-1-yl)-propylamin

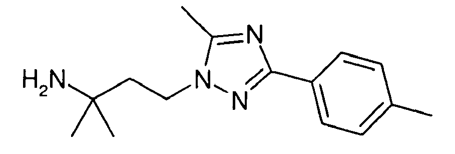

### a) 4-Methyl-benzoesäure-(1-imino-ethyl)-hydrazid

1.65 g (72 mmol) Natrium werden in 80 mL Ethanol gelöst. 8.89 g (72 mmol) Ethylacetimidat hydrochlorid in 160 mL Ethanol werden bei Raumtemperatur zugegeben und das ausfallende Natriumchlorid wird abfiltriert. Das Filtrat wird mit 6.00 g (40 mmol) 4-Methyl-benzoesäure hydrazid versetzt und über Nacht gerührt. Die Reaktionsmischung wird eingeengt und gekühlt. Der ausfallende Feststoff wird abfiltriert und mit kaltem Ethanol und Diethylether gewaschen (5.7 g weißer Feststoff). Aus dem Filtrat werden nach dem Abdestillieren der Lösungsmittel und Umkristallisation aus Ethanol weitere 1.2 g Feststoff gewonnen. Ausbeute: 6.93 g (91%); Massenspektroskopie [M+H]⁺ = 192.

### b) 5-Methyl-3-p-tolyl-[1,2,4]triazol

7.58 g (40 mmol) 4-Methyl-benzoesäure-(1-imino-ethyl)-hydrazid werden unter Rühren für 30 Minuten auf 180°C erwärmt. Nach Abkühlung wird der Feststoff in Chloroform gelöst. Der bei Kühlung ausfallende Niederschlag wird abgesaugt und aus Chloroform umkristallisiert. Ausbeute: 4.82 g (70%); Massenspektroskopie [M+H]⁺ = 174.

### c) [1,1-Dimethyl-3-(5-methyl-3-p-tolyl-[1,2,4]triazol-1-yl)-propyl]-carbaminsäure tert-butyl ester

Zu einer Lösung von 4.87 g (28 mmol) 5-Methyl-3-p-tolyl-[1,2,4]triazol in 40 mL DMPU werden bei 0°C 1.35 g (34 mmol, 60%ig) Natriumhydrid gegeben. Die Reaktionsmischung wird auf Raumtemperatur erwärmt und dann eine Stunde gerührt. 9.35 g (42 mmol) (3-Chlor-1,1-dimethyl-propyl)-carbaminsäure-tert-butylester und 1.87 g (5 mmol) Tetrabutylammoniumiodid werden zugegeben und es wird über Nacht bei Raumtemperatur und anschließend noch 2 Stunden bei 80°C gerührt. Man versetzt mit Wasser und Ethylacetat, trennt die wässrige Phase ab und extrahiert diese mit Ethylacetat. Die vereinigten organischen Phasen werden mit Wasser und Natriumchlorid-Lösung gewaschen, mit Natriumsulfat getrocknet und eingeengt. Der Rückstand wird mittels Säulenchromatographie gereinigt (Kieselgel; Petrolether/Ethylacetat = 1:1). Öl. Ausbeute: 2.97 g (30%); Massenspektroskopie [M+H]⁺ = 359.

### d) 1,1-Dimethyl-3-(5-methyl-3-p-tolyl-[1,2,4]triazol-1-yl)-propylamin

Zu eine Lösung von 2.97 g (8.3 mmol) [1,1-Dimethyl-3-(5-methyl-3-p-tolyl-[1,2,4]triazol-1-yl)-propyl]-carbaminsäure-tert-butyl ester in 80 mL Dichlormethan werden insgesamt 11 mL Trifluoressigsäure getropft und es wird über Nacht bei Raumtemperatur gerührt. Das Lösungsmittel wird abdestilliert und der Rückstand mit Diethylether versetzt und gerührt. Der ausfallende Feststoff wird abfiltriert und gewaschen. Ausbeute: 2.11 g (68%, Trifluoracetat); Massenspektroskopie [M+H]⁺ = 259.

### Zwischenprodukt 2: 3-[3-(4-Fluor-phenyl)-5-methyl-[1,2,4]triazol-1-yl]-1,1-dimethyl-propylamin

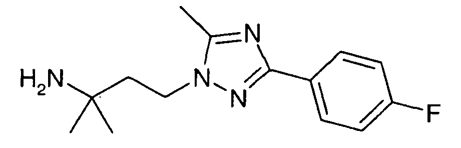

### a) 4-Fluor-benzoesäure-(1-imino-ethyl)-hydrazid

Hergestellt aus 7.2 g (58 mmol) Ethylacetimidat hydrochlorid und 5.00 g (32 mmol) 4-Fluor-benzoesäure hydrazid in Analogie zu dem für Zwischenprodukt 1a) beschriebenen Verfahren. Ausbeute: 5.78 g (91 %); Massenspektroskopie [M+H]⁺ = 196.

### b) 3-(4-Fluor-phenyl)-5-methyl-[1,2,4]triazol

Die-Darstellung erfolgt in Analogie zur Vorschrift für Zwischenprodukt 1b) aus 5.77 g (30 mmol) 4-Fluor-benzoesäure-(1-imino-ethyl)-hydrazid. Ausbeute: 4.11 g (78%); Massenspektroskopie [M+H]⁺ = 178.

### c) {3-[3-(4-Fluor-phenyl)-5-methyl-[1,2,4]triazol-1-yl]-1,1-dimethyl-propyl}-carbaminsäure tert-butyl ester

5.88 g (33 mmol) 3-(4-Fluor-phenyl)-5-methyl-[1,2,4]triazol werden in 40 mL DMPU gelöst und in der für Zwischenproduktl 1c) ausgeführten Weise mit 11.04 g (50 mmol) (3-Chlor-1,1-dimethyl-propyl)-carbaminsäure-tert-butylester, 1.59 g (40 mmol, 60%ig) Natriumhydrid und 2.21 g (6 mmol) Tetrabutylammoniumiodid umgesetzt. Ausbeute: 4.22 g (35%); Massenspektroskopie [M+H]⁺ = 363.

### d) 3-[3-(4-Fluor-phenyl)-5-methyl-[1,2,4]triazol-1-yl]-1,1-dimethyl-Propylamin

Erhalten aus der Umsetzung von 4.22 g (11.6 mmol) {3-[3-(4-Fluor-phenyl)-5-methyl-[1,2,4]triazol-1-yl]-1,1-dimethyl-propyl}-carbaminsäure-tert-butyl ester in 100 mL Dichlormethan und 15 mL Trifluoressigsäure. Weißer Feststoff. Ausbeute: 4.43 g (Trifluoracetat); Massenspektroskopie [M+H]⁺ = 263.

### Zwischenprodukt 3: 3-[3-(3,5-Difluor-phenyl)-5-methyl-[1,2,4]triazol-1-yl]-1,1-dimethyl-propylamin

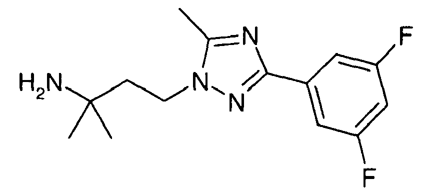

### a) 3,5-Difluor-benzoesäure-(1-imino-ethyl)-hydrazid

Aus 4.91 g (40 mmol) Ethylacetimidat hydrochlorid und 3.80 g (22 mmol) 3,5 Difluorbenzoesäure hydrazid in Analogie zur Vorschrift für Zwischenprodukt) 1a) erhalten. Ausbeute: 4.49 g (95%); Massenspektroskopie [M+H]⁺ = 214.

### b) 3-(3,5-Difluor-phenyl)-5-methyl-[1,2,4]triazol

Hergestellt aus 4.61 g (22 mmol) 3,5-Difluor-benzoesäure-(1-imino-ethyl)-hydrazid. Ausbeute: 3.81 g (91%); Massenspektroskopie [M+H]⁺= 196.

### c) {3-[3-(3,5-Difluor-phenyl)-5-methyl-[1,2,4]triazol-1-yl]-1,1-dimethyl-propyl}-carbaminsäure tert-butyl ester

3.74 g (19 mmol) 3-(3,5-Difluor-phenyl)-5-methyl-[1,2,4]triazol in 25 mL DMPU werden mit 0.92 g (23 mmol, 60%ig) Natriumhydrid, 6.37 g (29 mmol) (3-Chlor-1,1-dimethylpropyl)-carbaminsäure-tert-butylester und 1.27 g (3.5 mmol) Tetrabutylammoniumiodid in Analogie zu Zwischenprodukt 1c) umgesetzt. Öl. Ausbeute: 2.62 g (36%); Massenspektroskopie [M+H]⁺ = 381.

### d) 3-[3-(3,5-Difluor-phenyl)-5-methyl-[1,2,4]triazol-1-yl]-1,1-dimethyl-propylamin

2.62 g (6.9 mmol) {3-[3-(3,5-Difluor-phenyl)-5-methyl-[1,2,4]triazol-1-yl]-1,1-dimethylpropyl}-carbaminsäure-tert-butyl ester in 65 mL Dichlormethan werden mit 9 mL Trifluoressigsäure in der für Zwischenprodukt 1d) beschriebenen Weise umgesetzt. Weißer Feststoff. Ausbeute: 2.11 g (Trifluoracetat); Massenspektroskopie [M+H]⁺ = 281.

### Zwischenprodukt 4: 3-[5-Ethyl-3-(4-methoxy-phenyl)-[1,2,4]triazol-1-yl]-1,1-dimethyl-propylamin

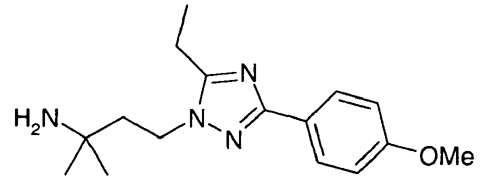

### a) 4-Methoxy-benzoesäure-(1-imino-propyl)-hydrazid

Herstellung aus 4.90 g (45 mmol) Propioamidin Hydrochlorid und 5.00 g (30 mmol) 4-Methoxy-benzoesäurehydrazid in Analogie zur Vorschrift für Zwischenprodukt 1a). Nach dem Abdestillieren des Ethanols werden 10.0 g Rohprodukt erhalten, die ohne weitere Aufreinigung umgesetzt werden.

### b) 5-Ethyl-3-(4-methoxy-phenyl)-[1,2,4]triazol

9.99 g (60%ig , ca. 28 mmol) 4-Methoxy-benzoesäure-(1-imino-propyl)-hydrazid werden für zwei Stunden auf 150°C erwärmt. Nach Abkühlung wird die Schmelze mittels Chromatographie an einer Kieselgelsäule (Petrolether/Ethylacetat = 3:7) gereinigt. Leicht gelber Feststoff. Ausbeute: 4.56 g (75% über zwei Stufen); Massenspektroskopie [M+H]⁺ = 204.

### c){3-[5-Ethyl-3-(4-methoxy-phenyl)-[1,2,4]triazol-1-yl]-1,1-dimethyl-propyl-carbaminsäure tert-butyl ester

4.30 g (21.2 mmol) 5-Ethyl-3-(4-methoxy-phenyl)-[1,2,4]triazol werden in 30 mL DMPU gelöst und auf 0°C gekühlt. Unter Schutzgasatmosphäre werden anschließend portionsweise 1.02 g (24 mmol, 60%ig) Natriumhydrid zugegeben und die Reaktionsmischung wird langsam auf Raumtemperatur erwärmt und dann eine Stunde gerührt. 6.10 g (27.5 mmol) (3-Chlor-1,1-dimethyl-propyl)-carbaminsäure-tert-butylester und 1.41 g (3.8 mmol) Tetrabutylammoniumiodid werden zugesetzt. Man läßt über Nacht rühren und beendet dann die Reaktion durch Zugabe von Wasser und Ethylacetat. Die wässrige Phase wird abgetrennt und mit Ethylacetat extrahiert. Die vereinigten organischen Phasen werden mit Natriumchlorid-Lösung gewaschen, mit Natriumsulfat getrocknet und eingeengt. Das zurückbleibende Öl wird mittels Chromatographie an einer Kieselgelsäule (Petrolether/Ethylacetat = 3:7) gereinigt. Ausbeute: 6.82 g (83%); Massenspektroskopie [M+H]⁺ = 389.

### d) 3-[5-Ethyl-3-(4-methoxy-phenyl)-[1,2,4]triazol-1-yl]-1,1-dimethyl-propylamin

Zu einer Lösung von 6.81 g (17.5 mmol) {3-[5-Ethyl-3-(4-methoxy-phenyl)-[1,2,4]triazol-1-yl]-1,1-dimethyl-propyl-carbaminsäure tert-butyl ester in 150 mL Dichlormethan werden insgesamt 20 mL Trifluoressigsäure getropft. Nach drei Stunden Rühren bei Raumtemperatur wird die Lösung eingeengt und das zurückbleibende Öl mit Diethylether versetzt. Der ausfallende weiße Feststoff wird abfiltriert mit Diethylether gewaschen und getrocknet. Ausbeute: 7.86 g (Trifluoracetat); Massenspektroskopie [M+H]⁺ = 289.

### Zwischenprodukt 5: 3-[1-(3-Amino-3-methyl-butyl)-5-methyl-1H-[1,2,4]triazol-3-yl]-benzoesäuremethlylester

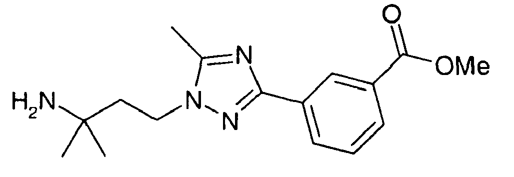

### a) 3-[N'-Benzyloxycarbonyl-hydrazinocarbonyl)-benzoesäuremethylester

Zu einer Lösung von 9.04 g (54.4 mmol) Hydrazincarbonsäurebenzylester in 100 mL Diethylether, 100 mL Dichlormethan und 4.83 mL Pyridin werden unter Kühlung mit einem Eisbad 10.80 g (54.4 mmol) 3-Chlorcarbonyl-benzoesäuremethylester in 100 mL Diethylether getropft. Die Reaktionsmischung wird über Nacht bei Raumtemperatur gerührt und dann mit Wasser versetzt. Der ausfallende Feststoff wird abfiltriert und mit Diethylether gewaschen. Weißer Feststoff. Ausbeute: 14.1 g (79%); Massenspektroskopie [M-H]⁺ = 327.

### b) 3-Hydrazinocarbonyl-benzoesäuremethylester

14.6 g (44.5 mmol) 3-[N'-Benzyloxycarbonyl-hydrazinocarbonyl)-benzoesäuremethylester werden in 75 mL Methanol gelöst und in Gegenwart von Palladium auf Kohle (10%ig) bei Raumtemperatur und 3 bar Wasserstoffdruck hydriert. Der Katalysator wird abfiltriert und das Filtrat vom Lösungsmittel befreit. Weißer Feststoff. Ausbeute: 7.98 g (92%); Massenspektroskopie [M+H]⁺ = 195.

### c) 3-[N'-(1-Imino-ethyl)-hydrazinocarbonyl]-benzoesäuremethylester

Hergestellt in Analogie zu dem für Zwischenprodukt 1a) beschriebenen Verfahren aus 3-Hydrazinocarbonyl-benzoesäuremethylester und Ethylacetimidat hydrochlorid. Weißer Feststoff. Ausbeute: 8.60 g (90%); Massenspektroskopie [M+H]⁺ = 236.

### d) 3-(5-Methyl-1H-[1.24]triazol-3-yl)-benzoesäuremethylester

8.10 g (34.4 mmol) 3-[N'-(1-Imino-ethyl)-hydrazinocarbonyl]-benzoesäuremethylester werden für 30 Minuten auf 180°C erwärmt. Zu dem nach Abkühlung vorliegenden Feststoff werden 80 mL Chloroform gegeben. Die Suspension wird filtriert und das Produkt getrocknet. Weißer Feststoff. Ausbeute: 4.03 g (55%); Massenspektroskopie [M+H]⁺ = 218.

### e) 3-[1-(3-tert-Butoxycarbonylamino-3-methyl-butyl)-5-methyl-1H-[1,2,4]triazol-3-yl-benzoesäuremethylester

6.00 g (27.6 mmol) 3-(5-Methyl-1H-[1,24]triazol-3-yl)-benzoesäuremethylester und 9.19 g (41.4 mmol) (3-Chlor-1,1-dimethyl-propyl)-carbaminsäure-tert-butylester werden in der für Zwischenprodukt 1c) beschriebenen Weise umgesetzt und aufgearbeitet. Gelbes Öl. Ausbeute: 5.96 g (54%); Massenspektroskopie [M+H]⁺ = 403.

### f) 3-[1-(3-Amino-3-methyl-butyl)-5-methyl-1H-[1,2 4]triazol-3-yl]-benzoesäuremethylester

Erhalten aus 3-[1-(3-tert-Butoxycarbonylamino-3-methyl-butyl)-5-methyl-1H-[1,2,4]triazol-3-yl-benzoesäuremethylester in Analogie zu dem für Zwischenprodukt 1d) beschriebenen Verfahren. Ausbeute: 5.36 g (68%, Ditrifluoracetat); Massenspektroskopie [M+H]⁺ = 303.

### Zwischenprodukt 6: 3-[1-(3-Amino-3-methyl-butyl)-5-methyl-1H-[1,2,4]triazol-3-yl]-benzoesäuremethylester

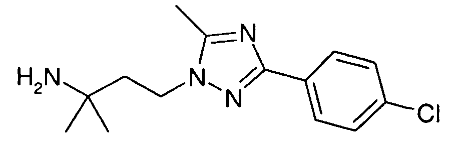

### a) 4-Chlor-benzoesäure N'-(1-imino-ethyl)-hydrazid

Zu einer Lösung von 1.91 g (20 mmol) Acetamidin hydrochlorid in 30 mL Ethanol werden 1.09 g (20 mmol) Natriummethylat in 20 mL Ethanol gegeben. Es wird 30 Minuten bei Raumtemperatur gerührt und dann filtriert. Das Filtrat wird mit 2.3 g (13.5 mmol) 4-Chlorbenzoesäure hydrazid versetzt, über Nacht bei Raumtemperatur gerührt, mit einem Eisbad gekühlt und dann filtriert. Der Niederschlag wird mit kalten Ethanol gewaschen und getrocknet. Gelber Feststoff. Ausbeute: 1.45 g (51 %); Massenspektroskopie (M+H]⁺= 212/214.

### b) 3-(4-Chlor-phenyl)-5-methyl-1H-[1,24]triazol

6.10 g (28.8 mmol) 4-Chlor-benzoesäure N'-(1-imino-ethyl)-hydrazid werden für 30 Minuten auf 180°C erwärmt. Nach Abkühlung werden aus dem Rückstand durch Umkristallisation in Chloroform 2.3 g Produkt erhalten. Einengen der Mutterlauge und anschließende Reinigung des Rückstands mittels Chromatographie (Kieselgel, Petrolether/Ethylacetat = 1:6) liefern zusätzliche 1.22 g Produkt. Weißer Feststoff. Ausbeute: 3.51 g (63%); Massenspektroskopie [M+H]⁺ = 194/196.

### c) {3-[3-(4-Chlor-phenyl)-5-methyl-[1,2,4]triazol-1-yl]-1,1-dimethyl-propyl}-carbaminsäure tert-butyl ester

3.48 g (18.0 mmol) 3-(4-Chlor-phenyl)-5-methyl-1H-[1,24]triazol und 5.98 g (27.0 mmol) (3-Chlor-1,1-dimethyl-propyl)-carbaminsäure-tert-butylester werden in der für Zwischenprodukt 1c) beschriebenen Weise umgesetzt und aufgearbeitet. Gelbes Öl. Ausbeute: 3.89 g (57%); Massenspektroskopie [M+H]⁺ = 379/381.

### d) 3-(1-(3-Amino-3-methyl-butyl)-5-methyl-1H-[1,2,41triazol-3-yl]-benzoesäuremethylester

Erhalten aus {3-[3-(4-Chlor-phenyl)-5-methyl-[1,2,4]triazol-1-yl]-1,1-dimethyl-propyl}-carbaminsäure-tert-butyl ester nach dem für Zwischenprodukt 1d) beschriebenen Verfahren. Ausbeute: 3.65 g (Trifluoracetat); Massenspektroskopie [M+H]⁺ = 279/281.

### Zwischenprodukt 7: 1,1-Dimethyl-3-[5-methyl-3-(4-trifluormethyl-phenyl)-[1,2,4]triazol-1-yl]-propylamin

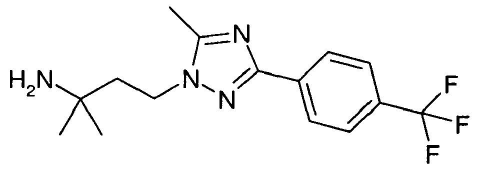

### a) 4-Trifluormethyl-benzoesäure N'-(1-imino-ethyl)-hydrazid

4.78 g (23.4 mmol) 4-(Trifluormethyl)benzoesäure hydrazid und 5.21 g (42.1 mmol) Ethylacetimidat hydrochlorid werden in der für Zwischenprodukt 1a) beschriebenen Weise umgesetzt. Ausbeute: 6.02 g; Massenspektroskopie [M+H]⁺ = 246.

### b) 5-Methyl-3-(4-trifluormethyl-phenyl)-1H-[1,2,4]triazol

Hergestellt aus 6.02 g (24.6 mmol) 4-Trifluormethyl-benzoesäure N'-(1-imino-ethyl)-hydrazid in Analogie zu dem für Zwischenprodukt 1b) beschriebenen Verfahren. Weißer Feststoff. Ausbeute: 4.76 g (85%); Massenspektroskopie [M+H]⁺ = 228.

### c) {1,1-Dimethyl-3-[5-methyl-3-(4-trifluormethyl-phenyl)-[1,2,4]triazol-1-yl]-propyl}-carbaminsäure-tert-butylester

Die Zielverbindung wird in Analogie zu dem für Zwischenprodukt 1c) beschriebenen Verfahren aus 4.90 g (21.6 mmol) 5-Methyl-3-(4-trifluormethyl-phenyl)-1H-[1,2,4]triazol und 7.17 g (32.4 mmol) (3-Chlor-1,1-dimethyl-propyl)-carbaminsäure-tert-butylester erhalten. Weißer Feststoff. Ausbeute: 5.06 g (57%); Massenspektroskopie [M+H]⁺ = 413.

### d) 1,1-Dimethyl-3-[5-methyl-3-(4-trifluormethyl-phenyl)-(1,2,41triazol-1-yl]-propylamin

Herstellung nach dem für Zwischenprodukt 1d) beschriebenen Verfahren aus {1,1-Dimethyl-3-[5-methyl-3-(4-trifluormethyl-phenyl)-[1,2,4]triazol-1-yl]-propyl}-carbaminsäure-tert-butylester. Weißer Feststoff. Ausbeute: 4.72 g (Trifluoracetat); Massenspektroskopie [M+H]⁺ = 313.

### Zwischenprodukt 8: 3-(3-Benzo[1,3]dioxo]-5-yl-5-methyl-[1,2,4]triazol-1-yl)-1,1-dimethyl-propylamin

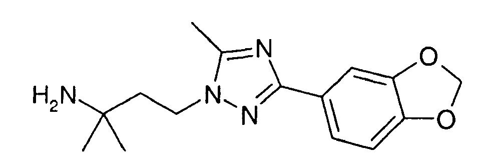

Die Herstellung erfolgt in Analogie zu den vorstehend beschriebenen Synthesen. Massenspektroskopie [M+H]⁺ = 289.

### Zwischenprodukt 9: 3-[3-(4-Methoxy-phenyl)-5-methyl-[1,2,4]triazol-1-yl]-1,1-dimethyl-propylamin

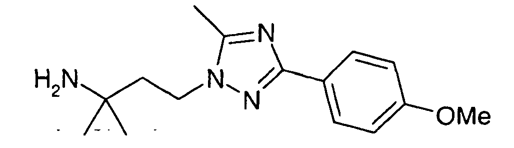

### a) 4-Methoxy-benzoesäure N'-(1-imino-ethyl)-hydrazid

4.6 g (0.20 mol) Natrium in 200 mL Ethanol werden bei Raumtemperatur mit einer Lösung von 25 g (0.20 mol) Ethylacetimidat hydrochlorid in 200 mL Ethanol versetzt. Das ausfallende Natriumchlorid wird abgesaugt und zum Filtrat werden 33.2 g (0.20 mol) 4-Methoxybenzoesäurehydrazid gegeben. Die Reaktionsmischung wird über Nacht bei Raumtemperatur gerührt und dann abgekühlt. Der ausfallende Niederschlag wird abgetrennt und mit Ethanol und Diethylether gewaschen. Ausbeute: 33.6 g (81 %); Schmelzbereich = 179-181°C.

### b) 3-(4-Methoxy-phenyl)-5-methyl-1H-[1,2,4]triazol

33.6 g(162 mmol) 4-Methoxy-benzoesäure N'-(1-imino-ethyl)-hydrazid werden für 30 Minuten auf 180°C erwärmt. Nach dem Abkühlen wird die erstarrte Schmelze in 250 mL Chloroform gelöst und wiederholt mit wässriger Natronlauge ausgeschüttelt. Die wässrigen Phasen werden vereinigt, mit Chloroform gewaschen, filtriert und durch Zugabe von Eisessig auf einen sauren pH-Wert eingestellt. Der ausfallende Feststoff wird abgesaugt, mit Wasser gewaschen und durch Erwärmen in Chloroform gelöst. Das Lösungsmittel wird eingeengt und der Rückstand filtriert. Der Feststoff wird mit Chloroform und Diethylether gewaschen. Ausbeute: 23.1 g (75%); Schmelzbereich = 169-171°C.

### c) 3-[3-(4-Methoxy-phenyl)-5-methyl-[1,2,4]triazol-1-yl]-1,1-dimethyl-propylamin

Die Zielverbindung wird aus der Umsetzung von 21.4 g (113 mmol) 3-(4-Methoxyphenyl)-5-methyl-1H-[1,2,4]triazol und 25 g (119 mmol) (3-Chlor-1,1-dimethyl-propyl)-[1-phenyl-methyliden]-amin erhalten. Das Produkt wird in 100 mL Aceton gelöst und mit 8.5 mL 32%iger wässriger Salzsäure angesäuert und gekühlt. Das ausfallende Hydrochlorid wird abgesaugt und mit Aceton und Diethylether gewaschen. Ausbeute: 20.3 g; Schmelzbereich = 190-194°C.

### Allgemeine Arbeitsvorschrift 1:

1 mmol 6-Benzyloxy-8-(2-ethoxy-2-hydroxy-acetyl)-4H-benzo[1,4]oxazin-3-on und 1 mmol Amin werden 15 Minuten in 5 mL Tetrahydrofuran bei 60°C gerührt. Man kühlt auf 0°C ab und tropft unter Argonatmosphäre 1.5 mL einer 2 molaren Lösung von Lithiumborhydrid in Tetrahydrofuran zu. Es wird 15 min bei 0°C gerührt, mit 10 mL Dichlormethan und 3 mL Wasser versetzt, eine weitere Stunde bei Raumtemperatur gerührt und dann über Kieselgur filtriert, wobei man mit Dichlormethan eluiert. Das Eluat wird vom Lösungsmittel befreit und der Rückstand, falls notwendig, chromatographisch gereinigt. Der so erhaltene Benzylether wird in Methanol gelöst und mit Palladium auf Kohle (10%ig) als Katalysator bei 2.5 bar und Raumtemperatur hydriert. Anschließend wird der Katalysator abgetrennt und das Rohprodukt mittels Chromatographie (Reverse Phase, Acetonitril/Wasser-Gradient mit 0.1 % Trifluoressigsäure) gereinigt.

### Allgemeine Arbeitsvorschrift 2:

1 mmol 6-Benzyloxy-8-(2-ethoxy-2-hydroxy-acetyl)-4H-benzo[1,4]oxazin-3-on und 1 mmol Amin werden in 5 mL Ethanol suspendiert und auf 70°C erwärmt. Die entstandene Lösung wird eine Stunde bei 70°C gerührt und dann auf Raumtemperatur abgekühlt. Nach Zugabe von 113 mg (3 mmol) Natriumborhydrid wird 3 Stunden bei Raumtemperatur gerührt, mit 0.7 mL gesättigter Kaliumcarbonatlösung versetzt und weitere 30 Minuten gerührt. Es wird über Aluminiumoxid (basisch) filtriert, wiederholt mit Dichlormethan/Methanol = 15:1 nachgewaschen, eingeengt und chromatographiert (Kieselgel; Dichlormethan mit 0-10% Methanol :Ammoniak = 9:1). Der so erhaltene Benzylether wird in 10 mL Methanol gelöst und mit Palladium auf Kohle als Katalysator bei 1 bar Wasserstoffdruck hydriert. Anschließend wird der Katalysator abfiltriert und das Filtrat eingeengt.

### Beispiel 1: 8-(2-{3-[3-(4-Fluorphenyl)-5-methyl-[1,2,4]triazol-1-yl]-1,1-dimethyl-propylamino}-1-hydroxy-ethyl)-6-hydroxy-4H-benzo[1,4]oxazin-3-on

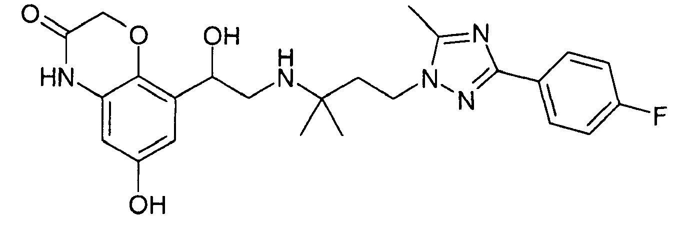

Erhalten aus der Umsetzung von 6-Benzyloxy-8-(2-ethoxy-2-hydroxy-acetyl)-4H-benzo[1,4]oxazin-3-on und 3-[3-(4-Fluor-phenyl)-5-methyl-[1,2,4]triazol-1-yl]-1,1-dimethyl-propylamin nach der allgemeinen Arbeitsvorschrift 1. Die abschließende Reinigung erfolgt mittels Chromatographie (Reverse Phase, Acetonitril/Wasser-Gradient mit 0.1% Trifluoressigsäure). Weißer Feststoff. Ausbeute: 134 mg (29%, Trifluoracetat); Massenspektroskopie [M+H]⁺ = 470.

### Beispiel 2: 8-{2-[1,1-Dimethyl-3-(5-methyl-3-p-tolyl-[1,2,4]triazol-1yl)-propylamino]-1-hydroxy-ethyl}-6-hydroxy-4H-benzo[1,4]oxazin-3-on

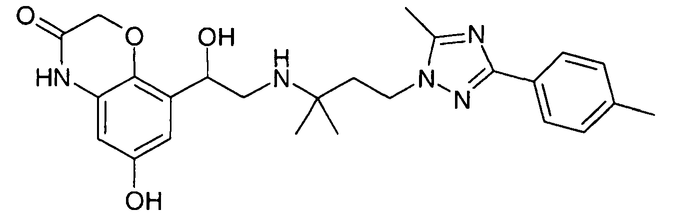

Hergestellt aus 6-Benzyloxy-8-(2-ethoxy-2-hydroxy-acetyl)-4H-benzo[1,4]oxazin-3-on und 1,1-Dimethyl-3-(5-methyl-3-p-tolyl-[1,2,4]triazol-1-yl]-propylamin nach der allgemeinen Arbeitsvorschrift 1. Hellbeiger Feststoff. Ausbeute: 283 mg (49%, Trifluoracetat); Massenspektroskopie [M+H]⁺ = 466.

### Beispiel 3: 8-(2-{1,1-Dimethyl-3-[5-methyl-3-(4-trifluormethyl-phenyl)-[1,2,4]triazol-1-yl]-propylamino}-1-hydroxy-ethyl)-6-hydroxy-4H-benzo[1,4]oxazin-3-on

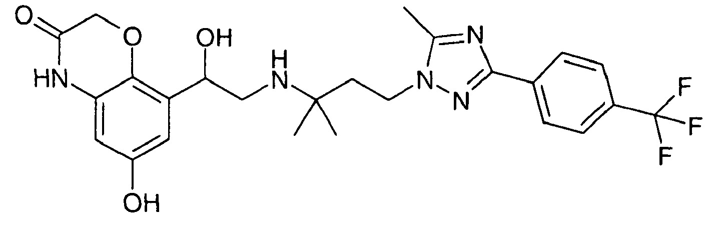

Hergestellt aus 6-Benzyloxy-8-(2-ethoxy-2-hydroxy-acetyl)-4H-benzo[1,4]oxazin-3-on und 1,1-Dimethyl-3-[5-methyl-3-(4-trifluormethyl-phenyl)-[1,2,4]triazol-1-yl]-propylamin in Analogie zur allgemeinen Arbeitsvorschrift 1. Beiger Feststoff. Ausbeute: 234 mg (37%, Trifluoracetat); Massenspektroskopie [M+H]⁺ = 520.

### Beispiel 4: 8-(2-{3-[3-(3,5-Difluor-phenyl)-5-methyl-[1,2,4]triazol-1-yl]-1,1-dimethyl-propylamino}-1-1-hydroxy-ethyl)-6-hydroxy-4H-benzo[1,4]oxazin-3-on

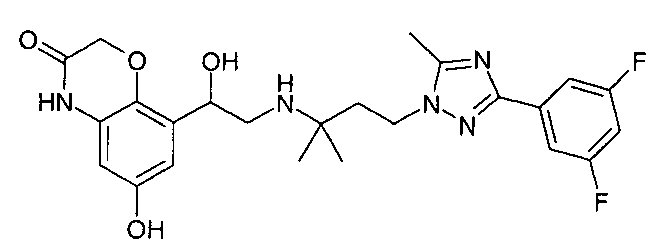

Hergestellt aus 6-Benzyloxy-8-(2-ethoxy-2-hydroxy-acetyl)-4H-benzo[1,4]oxazin-3-on und 3-[3-(3,5-Ditluor-phenyl)-5-methyl-[1,2,4]triazol-1-yl]-1,1-dimethyl-propylamin nach der allgemeinen Arbeitsvorschrift 1. Weißer Feststoff. Ausbeute: 208 mg (35%, Trifluoracetat); Massenspektroskopie [M+H]⁺ = 488.

### Beispiel 5: 3-(1-{3-[2-Hydroxy-2-(6-hydroxy-3-oxo-3.4-dihydro-2H-benzo[1,4]oxazin-8-yl)-ethylamino]-3-methyl-butyl}1-5-methyl-1H-[1,2,4]triazol-3-yl-benzoesäure

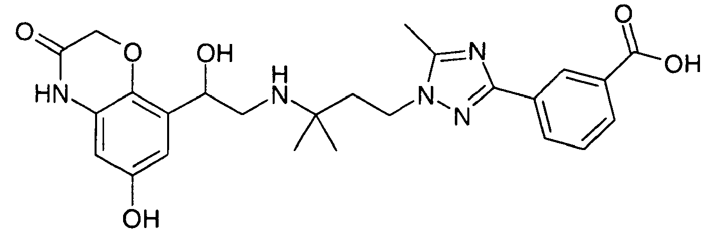

### a) 3-(1-{3-[2-(6-Benzyloxy-3-oxo-3,4-dihydro-2H-benzo[1,4]oxazin-8-yl)-2-hydroxy-ethylamino]-3-methyl-butyl}-5-methyl-1H-[1,2,4]triazol-3-yl)-benzoesäuremethylester

Hergestellt aus 6-Benzyloxy-8-(2-ethoxy-2-hydroxy-aceiyl)-4H-benzo[1,4]oxazin-3-on und 3-[1-(3-Amino-3-methyl-butyl)-5-methyl-1H-[1,2,4]triazol-3-yl]-benzoesäuremethylester in Analogie zur allgemeinen Arbeitsvorschrift 1. Die abschließende Reinigung erfolgt mittels Chromatographie (Reverse Phase, Acetonitril/Wasser-Gradient mit 0.1 % Trifluoressigsäure). Ausbeute: 550 mg (77%, Trifluoracetat); Massenspektroskopie [M+H]⁺= 510.

### b) 3(1-{3-[2-(6-Benzyloxy-3-oxo-3,4-dihydro-2H-benzo[1,4]oxazin-8-yl)-2-hydroxy-ethylamino]-3-methyl-butyl]-5-methyl-1H-[1,2,4]triazol-3-yl)-benzoesäure

Eine Lösung von 550 mg (0.72 mmol) 3-(1-{3-[2-(6-Benzyloxy-3-oxo-3,4-dihydro-2H-benzo[1,4]oxazin-8-yl)-2-hydroxy-ethylamino]-3-methyl-butyl -5-methyl-1H-[1,2,4]triazol-3-yl)-benzoesäuremethylester trifluoracetat in 10 mL Methnol wird mit 2 mL einer 2 molare Natriumhydroxid-Lösung versetzt und 30 Minuten unter Rückfluß erwärmt. Nach dem Abdestillieren des Methanols werden 5 mL Wasser, 10 mL n-Butanol und 5 mL Essigsäure zugegeben. Der ausfallende Niederschlag wird abgesaugt und mit Diethylether gewaschen. Brauner Feststoff. Ausbeute: 300 mg (56%, Trifluoracetat); Massenspektroskopie [M+H]⁺ = 586.

### c) 3-(1-{3-(2-Hydroxy-2-(6-hydroxy-3-oxo-3,4-dihydro-2H-benzo[1,4]oxazin-8-yl)-ethylaminol-3-methyl-butyl}-5-methyl-1H-[1,2,4]triazol-3-yl-benzoesäure

250 mg (0.36 mmol) 3-(1-{3-[2-(6-Benzyloxy-3-oxo-3,4-dihydro-2H-benzo[1,4]oxazin-8-yl)-2-hydroxy-ethylamino]-3-methyl-butyl}-5-methyl-1H-[1,2,4]triazol-3-yl)-benzoesäure trifluoracetat werden in 5 mL Methanol gelöst und in Gegenwart von Palladium auf Kohle (10%ig) bei Raumtemperatur und 2.5 bar Wasserstoffdruck hydriert. Der Katalysator wird abgesaugt, das Filtrat eingeengt und der Rückstand chromatographisch gereinigt (Reverse Phase, Acetonitril/Wasser-Gradient mit 0.1% Trifluoressigsäure). Gelber Feststoff. Ausbeute: 62 mg (28%, Trifluoracetat); Massenspektroskopie [M+H]⁺ = 496.

### Beispiel 6: 8-(2-{3-[3-(4-Chlor-phenyl)-5-methyl-[1,2,4]triazol-1-yl]-1,1-dimethyl-propylamino}-1-hydroxy-ethyl)-6-hydroxy-4H-benzo[1,4]oxazin-3-on

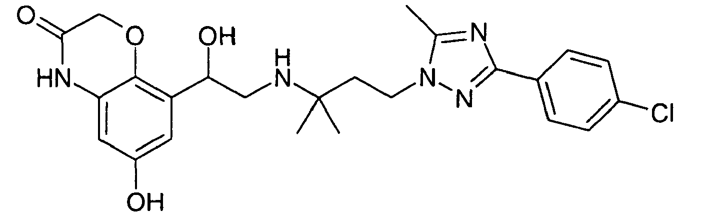

### a) 6-Benzyloxy-8-(2-{3-[3-(4-chlor-phenyl)-5-methyl-[1,2,4]triazol-1-yl]-1,1-dimethyl-propylamino}1-1-hydroxy-ethyl)-4H-benzo[1,4]oxazin-3-on

Hergestellt aus 6-Benzyloxy-8-(2-ethoxy-2-hydroxy-acetyl)-4H-benzo[1,4]oxazin-3-on und 3-[3-(4-Chlor-phenyl)-5-methyl-[1,2,4]triazol-1-yl]-1,1-dimethyl-propylamin in Analogie zur allgemeinen Arbeitsvorschrift 1. Das Rohprodukt wird mittels Chromatographie (Reverse Phase, Acetonitril/Wasser-Gradient mit 0.1% Trifluoressigsäure) gereinigt. Ausbeute: 550 mg (80%, Trifluoracetat); Massenspektroskopie [M+H]⁺ = 576.

### 8-(2-{3-[3-(4-Chlor-phenyl)-5-methyl-[1,2,4]triazol-1-yl]-1,1-dimethyl-propylamino}-1-hydroxy-ethyl)-6-hydroxy-4H-benzo[1,4]oxazin-3-on

550 mg (0.80 mmol) 6-Benzyloxy-8-(2-{3-[3-(4-chlor-phenyl)-5-methyl-[1,2,4]triazol-1-yl]-1,1-dimethyl-propylamino)-1-hydroxy-ethyl)-4H-benzo[1,4]oxazin-3-on werden in 3

mL Dichlormethan gelöst und auf -78°C abgekühlt. 2 mL einer 1 molaren Lösung von Bortribromid in Dichlormethan werden zugetropft und es wird auf Raumtemperatur erwärmt. Man läßt 10 Minuten bei dieser Temperatur Rühren und versetzt dann mit 10 mL Dichlormethan und 3 mL Wasser und rührt 30 Minuten. Es wird über Kieselgur filtriert, wobei man mit Dichlormethan und Methanol eluiert. Das Eluat wird eingeengt und der Rückstand mittels Chromatographie (Reverse Phase, Acetonitril/Wasser-Gradient mit 0.1% Trifluoressigsäure) gereinigt. Weißer Feststoff. Ausbeute: 29 mg (6%, Trifluoracetat); Massenspektroskopie [M+H]⁺ = 486/8.

### Beispiel 7: 8-(2-{3-[5-Ethyl-3-(4-methoxy-phenyl)-[1,2,4]triazol-1-yl]-1,1-dimethyl-propylamino}-1-hydroxy-ethyl)-6-hydroxy-4H-benzo[1,4]oxazin-3-on

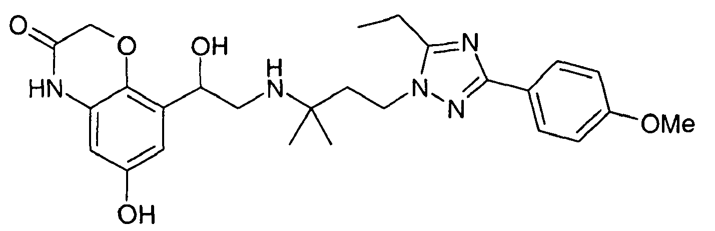

Hergestellt aus 6-Benzyloxy-8-(2-ethoxy-2-hydroxy-acetyl)-4H-benzo[1,4]oxazin-3-on und 3-[5-Ethyl-3-(4-methoxy-phenyl)-[1,2,4]triazol-1-yl]-1,1-dimethyl-propylamin nach der allgemeinen Arbeitsvorschrift 1. Ausbeute: 267 mg (44%, Trifluoracetat); Massenspektroskopie [M+H]⁺ = 496.

### Beispiel 8: 6-Hydroxy-8-(1-hydroxy-2-{3-[3-(4-methoxy-phenyl)-5-methyl-[1,2,4]triazol-1-yl]-1,1-dimethyl-propylamino}-ethyl)-4H-benzo[1,4]oxazin-3-on

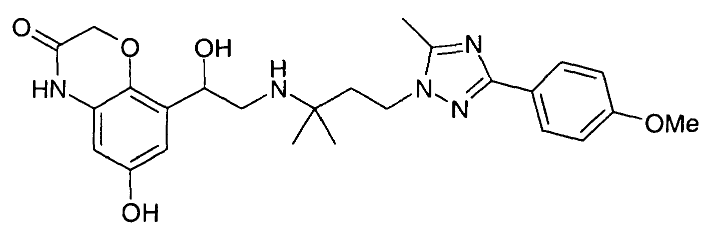

Hergestellt aus 6-Benzyloxy-8-(2-ethoxy-2-hydroxy-acetyl)-4H-benzo[1,4]oxazin-3-on und 3-[3-(4-Methoxy-phenyl)-5-methyl-[1,2,4]triazol-1-yl]-1,1-dimethyl-propylamin nach der allgemeinen Arbeitsvorschrift 2. Ausbeute: 217 mg (45%); Massenspektroskopie [M+H]⁺ = 482.

### Bespiel 9: 8-{2-[3-(3-Benzo[1,3]dioxol-5-yl-5-methyl-[1,2,4]triazol-1-yl)-1,1-dimethyl-propylaminol-1-hydroxy-ethyl}-6-hydroxy-4H-benzo[1,4]oxazin-3-on

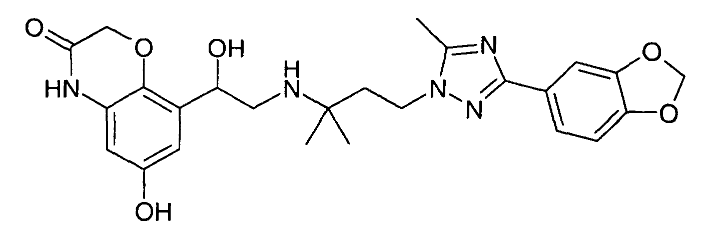

Hergestellt aus 6-Benzyloxy-8-(2-ethoxy-2-hydroxy-acetyl)-4H-benzo[1,4]oxazin-3-on und 3-(3-Benzo[1,3]dioxol-5-yl-5-methyl-[1,2,4]triazol-1-yl)-1,1-dimethyl-propylamin nach der allgemeinen Arbeitsvorschrift 2. Ausbeute: 236 mg (48%); Massenspektroskopie [M+H]⁺ = 496.

### Beispiel 10: 7-{2-[3-(3-Benzo[1,3]dioxol-5-yl-5-methyl-[1,2,4]triazol-1-yl)-1,1-dimethyl-propylamino]1-hydroxy-ethyl}-5-hydroxy-3H-benzooxazol-2-on

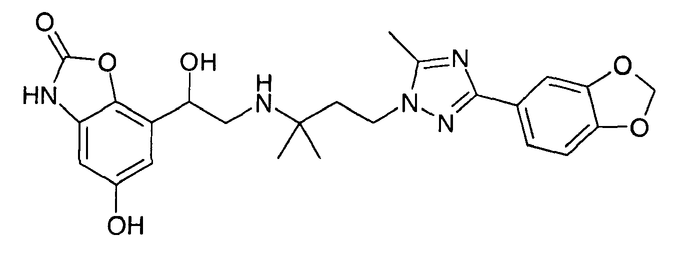

### a) 7-Acetyl-5-benzyloxy-3H-benzooxazol-2-on

In eine Lösung von 121 g (0.47 mol) 1-(3-Amino-5-benzyloxy-2-hydroxy-phenyl)-ethanon in 800 mL Pyridin werden bei 20 bis 40°C 52 g (0.53 mol) Phosgen eingeleitet. Die Reaktionsmischung wird für 2 Stunden auf 50°C erwärmt, dann auf Eis gegossen und mit konz. Salzsäure angesäuert. Es wird ein rotbrauner Feststoff isoliert, der wiederholt in Ethanol unter Zusatz von Aktivkohle umkristallisiert wird. Ausbeute: 67.5 g (51%); Schmelzbereich: 163-166°C.

### b) 5-Benzyloxy-7-(2-ethoxy-2-hydroxy-acetyl)-3H-benzooxazol-2-on

20 g (71 mmol) 7-Acetyl-5-benzyloxy-3H-benzooxazol-2-on und 8 g (72 mmol) Selendioxid werden in Gegenwart von Aktivkohle in 100 mL Dioxan und 3.1 mL Wasser über 8 Stunden unter Rückfluß gerührt. Der Feststoff wird abfiltriert, das Lösungsmittel abdestilliert und der Rückstand mit 50 mL Ethanol versetzt. Man lässt 15 Minuten refluxieren und filtriert dann über Aktivkohle. Der beim Abkühlen ausfallende Feststoff wird nach 3 Stunden abgesaugt und mit Ethanol und Diethylether gewaschen. Ausbeute: 7 g (29%); Schmelzbereich: 140-143°C.

### c) 17-{2-[3-(3-Benzo[1,3]dioxol-5-yl-5-methyl-[1,2,4]triazol-1-yl)-1,1-dimethyl-propylaminol-l-hydroxy-ethyll-5-hydroxy-3H-benzooxazol-2-on

72 mg (0.5 mmol) 5-Benzyloxy-7-(2-ethoxy-2-hydroxy-acetyl)-3H-benzooxazol-2-on und 144 mg (0.5 mmol) 3-(3-Benzo[1,3]dioxol-5-y1-5-methyl-[1,2,4]triazol-1-yl)-1,1-dimethyl-propylamin werden in 8 mL Ethanol 90 Minuten bei 80°C gerührt. Nach Abkühlung auf Raumtemperatur werden 19 mg (0.5 mmol) Natriumborhydrid zugesetzt und man lässt 2 Stunden bei Raumtemperatur Rühren. Es wird mit 1 N Salzsäure angesäuert, 10 Minuten gerührt und dann mit Kaliumcarbonatlösung alkalisch gestellt. Man verdünnt mit Ethylacetat und filtriert über Kieselgur, wobei die wässrigen Bestandteile abgetrennt werden. Die verbleibende organische Phase wird eingeengt und der Rückstand chromatographisch gereinigt. Der so erhaltene Benzylether wird in Ethanol gelöst und mit Palladium auf Kohle (10%ig) als Katalysator bei 2.5 bar und Raumtemperatur hydriert. Anschließend wird der Katalysator abgetrennt und das Rohprodukt mittels Chromatographie (Reverse Phase, Acetonitril/Wasser-Gradient mit 0.1% Trifluoressigsäure) gereinigt. Ausbeute: 8 mg (3%, Trifluoracetat); Massenspektroskopie [M+H]⁺ = 482.

### Beispiel 11: 8-{2-[3-(3-Benzo[1,3]dioxol-5-yl-5-methyl-[1,2,4]triazol-1-yl)-1,1-dimethyl-propylaminol-1-hydroxy-ethyl}-6-hydroxy-2.2-dimethyl-4H-benzo[1,4]oxazin-3-on

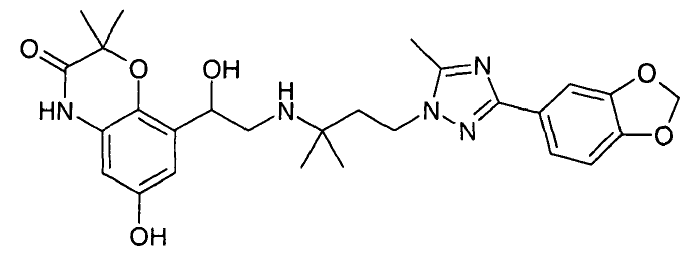

### a) N-(3-Acetyl-5-benzyloxy-2-hydroxy-phenyl)-2-brom-2-methyl-propionamid

Zu einer Lösung von 5.15 g (20 mmol) 1-(3-Amino-5-benzyloxy-2-hydroxy-phenyl)-ethanon in 20 mL Pyridin werden bei 5-20°C 4.64 g (25 mmol) 2-Brom-2-methyl-propionylchlorid getropft. Nach beendeter Zugabe wird 15 Minuten gerührt, mit Eiswasser und 100 mL Ethylacetat versetzt und mit konz. Salzsäure angesäuert. Die organische Phase wird abgetrennt, mit Wasser gewaschen und mit Natriumsulfat getrocknet. Nach dem Abdestillieren des Lösungsmittels wird der Rückstand aus einem Diethylether/Petrolether-Gemisch kristallisiert. Ausbeute: 6.8 g (84%); Schmelzbereich: 88-90°C.

### b) 8-Acetyl-6-benzyloxy-2,2-dimethyl-4H-benzo[1,4]oxazin-3-on

6.60 g (16.2 mmol) N-(3-Acetyl-5-benzyloxy-2-hydroxy-phenyl)-2-brom-2-methyl-propionamid und 2.76 g (20 mmol) Kaliumcarbonat werden 1 Stunde in 70 mL Acetonitril unter Rückfluß gerührt. Der Feststoff wird abgesaugt, das Filtrat eingeengt und der Rückstand mit 30 mL Ethylacetat versetzt. Nach erneuter Filtration und dem Abdestillieren des Lösungsmittels wird das Rohprodukt aus wenig Methanol kristallisiert. Ausbeute: 1.00 g (19%); Massenspektroskopie [M+H]⁺ = 326; Schmelzbereich = 148-150°C.

### c) 6-Benzyloxy-8-(2-ethoxy-2-hydroxy-acetyl)-2,2-dimethyl-4H-benzo[1,4]oxazin-3-on

Die Darstellung erfolgt in Analogie zu dem für die Beispiel 10b) beschriebenen Verfahren aus 8-Acetyl-6-benzyloxy-2,2-dimethyl-4H-benzo[1,4]oxazin-3-on.

### d) 8-{2-3-[3-Benzo[1,3]dioxol-5-yl-5-methyl-[1,2,4]triazol-1-yl)-1,1-dimethyl-propylaminol-1-hydroxy-ethyl}-6-hydroxy-2,2-dimethyl-4H-benzo[1,4]oxazin-3-on

Hergestellt aus 385 mg (1 mmol) 6-Benzyloxy-8-(2-ethoxy-2-hydroxy-acetyl)-2,2-dimethyl-4H-benzo[1,4]oxazin-3-on und 402 mg (1 mmol) 3-(3-Benzo[1,3]dioxol-5-yl-5-methyl-[1,2,4]triazol-1-yl)-1,1-dimethyl-propylamin nach der allgemeinen Arbeitsvorschrift 1. Ausbeute: 37 mg (6%, Trifluoracetat); Massenspektroskopie [M+H]⁺= 524.

Geeignete Anwendungsformen zur Applikation der Verbindungen der Formel **1** sind beispielsweise Tabletten, Kapseln, Zäpfchen, Lösungen, Pulver etc. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können. Säfte der erfindungsgemäßen Wirkstoffe können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösungsmittel als Lösevermittler bzw. Hilfslösungsmittel eingesetzt werden können, hergestellt und in Injektionsflaschen oder Ampullen oder Infusionsflaschen abgefüllt.

Die eine oder mehrere Wirkstoffe enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide), synthetische Gesteinsmehle (z.B. hochdisperse Kieselsäure und Silikate), Zucker-(z.B.Rohr-, Milch-und Traubenzuker), Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Bei der erfindungsgemäßen Applikation der Verbindungen der Formel **1** zur Therapie von Atemwegeserkrankungen werden besonders bevorzugt inhalativ applizierbare Darreichungsformen bzw. pharmazeutische Formulierungen eingesetzt. Als inhalierbare Darreichungsformen kommen Inhalationspulver, treibgashaltige Dosieraerosole oder treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfaßt. Die im Rahmen der vorliegenden Erfindung einsetzbaren Darreichungsformen werden im nachfolgenden Teil der Beschreibung detailliert beschrieben.

Erindungsgemäß einsetzbare Inhalationspulver können **1** entweder allein oder im Gemisch mit geeigneten physiologisch unbedenkliche Hilfsstoffen enthalten.

Sind die Wirkstoffe **1** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfsstoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhalationspulver mikronisierter Wirkstoff **1**, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen Inhalationspulver durch Mahlen und Mikronisieren sowie durch abschließendes Mischen der Bestandteile sind aus dem Stand der Technik bekannt.

Die erfindungsgemäßen Inhalationspulver können mittels aus dem Stand der Technik bekannten Inhalatoren appliziert werden.

Erfindungsgemäße treibgashaltige Inhalationsaerosole können **1** im Treibgas gelöst oder in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationsaerosole können mittels im Stand der Technik bekannten Inhalatoren (MDIs = metered dose inhalers) appliziert werden.

Ferner kann die Applikation der erfindungsgemäßen Wirkstoffe **1** in Form von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die **1** enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure und Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

In diesen Formulierungen kann gegebenenfalls auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg / 100 ml, bevorzugt unter 50 mg/100ml, besonders bevorzugt unter 20mg/100ml. Generell sind solche Inhalationslösungen bevorzugt, in denen der Gehalt an Natriumedetat bei 0 bis 10mg/100ml liegt.

Den treibgasfreien Inhalationslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besonders bevorzugt zwischen 5 und 20 mg/100ml enthalten.

Bevorzugte Formulierungen enthalten außer dem Lösungsmittel Wasser und dem Wirkstoff **1** nur noch Benzalkoniumchlorid und Natriumedetat.

In einer anderen bevorzugten Ausführungsform wird auf Natriumedetat verzichtet.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel **1** bereits bei Dosen im µg-Bereich durch eine hohe Wirksamkeit aus. Auch oberhalb des µg-Bereichs, lassen sich die Verbindungen der Formel **1** sinnvoll einsetzen. Die Dosierung kann dann beispielsweise auch im Milligrammbereich liegen.

Ein weiterer Aspekt der vorliegenden Erfindung betrifft die vorstehend genannten pharmazeutischen Formulierungen, gekennzeichnet durch einen Gehalt einer Verbindung der Formel **1**, als solche, besonders bevorzugt die vorstehend genannten inhalativ applizierbaren pharmazeutischen Formulierungen.

Die nachfolgenden Formulierungsbeipiele illustrieren die vorliegende Erfindung ohne sie jedoch in ihrem Umfang zu beschränken:

| | | |
|---|---|---|
| A) | Ampullenlösung | |
| | Wirkstoff der Formel **1** | 25 mg |
| | Natriumchlorid | 50 mg |
| | Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| | | |
|---|---|---|
| B) | Dosieraerosol (Suspension) | |
| | Wirkstoff der Formel **1** | 0.3 Gew.% |
| | Sorbitantrioleat | 0.6 Gew. % |
| | HFA134A:HFA227 2:1 | 99.1 Gew.% |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden

| | | |
|---|---|---|
| C) | Dosieraerosol (Lösung) | |
| | Wirkstoff der Formel **1** | 0.3 Gew.% |
| | Ethanol abs. | 20 Gew.% |
| | wässrige HCl 0.01 mol/l | 2.0 Gew.% |
| | HFA134A | 77.7 Gew.% |

Die Herstellung der Lösung erfolgt in üblicher Art und Weise durch Mischen der einzelnen Bestandteile.

| | | | |
|---|---|---|---|
| D) | Inhalationspulver | | |
| | Wirkstoff der Formel **1** | | 80 µg |
| | Lactose Monohydrat | ad | 10 mg |

Die Herstellung des Inhalationspulvers erfolgt in üblicher Art und Weise durch Mischen der einzelnen Bestandteile.

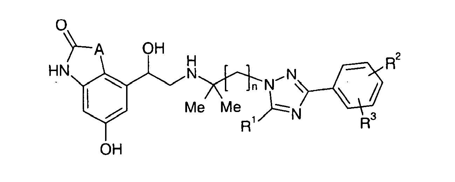

## Patentansprüche

1. Verbindungen der Formel **1**, 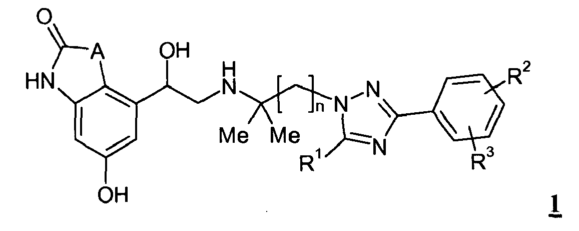 worin
n 1, 2 oder 3, bevorzugt 2;
A eine zweibindige Gruppe ausgewählt aus der Gruppe bestehend aus -CR⁴R⁵-O-, -CH=CH- oder -CH₂-CH₂-, bevorzugt -CR⁴R⁵-O-;
R¹ -C₁₋₄-Alkyl;
R² und R³, gleich oder verschieden H, C₁₋₄-Alkyl, C₂₋₄-Alkenyl, C₂₋₄-Alkinyl, C₃₋₆-Cycloalkyl, C₁₋₄-Haloalkyl, -O-C₁₋₄-Haloalkyl, Halogen, OH, CN, NO₂, -C₂₋₄-Alkyl-OH, -O-C₁₋₄-Alkyl, COOH oder COO-C₁₋₄-Alkyl, oder R² und R³ gemeinsam eine 2-bindige Gruppe ausgewählt aus -O-CR⁴R⁵-O-, -O-CR⁴R⁵-NR⁶- oder -CH=CH-CH=CH-;
R⁴ H oder C₁₋₄-Alkyl;
R⁵ H oder C₁₋₄-Alkyl;
R⁶ H oder C₁₋₄-Alkyl,
bedeuten, gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

2. Verbindungen der Formel **1** nach Anspruch 1,
worin
A eine zweibindige Gruppe ausgewählt aus der Gruppe bestehend aus -CR⁴R⁵-O-, -CH=CH- oder -CH₂-CH₂-, bevorzugt -CR⁴R⁵-O- mit
R⁴ H, Methyl, Ethyl, bevorzugt H oder Methyl, besonders bevorzugt H;
R⁵ H, Methyl, Ethyl, bevorzugt H oder Methyl, besonders bevorzugt H;
bedeuten, gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

3. Verbindungen der Formel **1** nach Anspruch 1 oder 2 , worin R¹ Methyl, Ethyl oder Propyl bedeutet, gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

4. Verbindungen der Formel **1** nach Anspruch 1, 2 oder 3, worin
R² H, Methyl, Ethyl, Propyl, Vinyl, Allyl, Propargyl, Cyclopropyl, Cyclopentyl, Cyclohexyl, -CH₂Cl, -CHCl₂, -CCl₃, -CH₂F, -CHF₂, -CF₃, -CH₂-CH₂Cl, -CH₂-CHCl₂, -CH₂-CCl₃, -CH₂-CH₂F, -CH₂-CHF₂, -CH₂-CF₃, -CH₂-CH₂OH, Fluor, Chlor, Brom, OH, CN, NO₂, Methoxy, Ethoxy, Propoxy, COOH, COO-Methyl, COO-Ethyl, COO-Propyl oder COO-Butyl;
R³ Methyl, Ethyl, Propyl, Vinyl, Allyl, Propargyl, Cyclopropyl, Cyclopentyl, Cyclohexyl, -CH₂Cl, -CHCl₂, -CCl₃, -CH₂F, -CHF₂, -CF₃, -CH₂-CH₂Cl, -CH₂-CHCl₂, -CH₂-CCl₃, -CH₂-CH₂F, -CH₂-CHF₂, -CH₂-CF₃, -CH₂-CH₂OH, Fluor, Chlor, Brom, OH, CN, NO₂, Methoxy, Ethoxy, Propoxy, COOH, COO-Methyl, COO-Ethyl, COO-Propyl oder COO-Butyl, oder R² und R³ gemeinsam eine 2-bindige Gruppe ausgewählt aus -O-CR⁴R⁵-O-, -O-CR⁴R⁵-NR⁶- oder -CH=CH-CH=CH-; R⁴ H, Methyl, Ethyl, bevorzugt H oder Methyl, besonders bevorzugt H;
R⁵ H, Methyl, Ethyl, bevorzugt H oder Methyl, besonders bevorzugt H;
R⁶ H, Methyl, Ethyl, bevorzugt H oder Methyl, besonders bevorzugt H;
bedeuten, gegebenenfalls in Form der einzelnen Enantiomere, Mischungen der einzelnen Enantiomere oder Racemate, gegebenenfalls in Form ihrer pharmakologisch unbedenklichen Säureadditionssalze, sowie gegebenenfalls in Form ihrer Solvate und/oder Hydrate.

5. Verbindungen der Formel **1** nach einem der Ansprüche 1 bis 4, **dadurch gekennzeichnet, dass** sie in Form eines ihrer Säureadditionssalze mit pharmakologisch unbedenklichen Säuren vorliegen, welches ausgewählt ist aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat.

6. Verbindungen der Formel **1** nach einem der Ansprüche 1 bis 5, **dadurch gekennzeichnet, dass** sie in Form der R-Enantiomere der Formel ***R*-1** 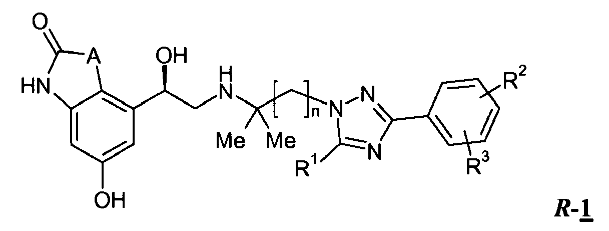 vorliegen.

7. Verbindungen der Formel **1** gemäß einem der Ansprüche 1 bis 6 als Arzneimittel.

8. Verwendung der Verbindungen der Formel **1** gemäß einem der Ansprüche 1 bis 6 zur Herstellung eines Arzneimittels zur Behandlung von Atemwegserkrankungen.

9. Pharmazeutische Formulierung, **gekennzeichnet durch** einen Gehalt einer Verbindung der Formel **1** gemäß einem der Ansprüche 1 bis 6.

## Claims

1. Compounds of formula **1**, 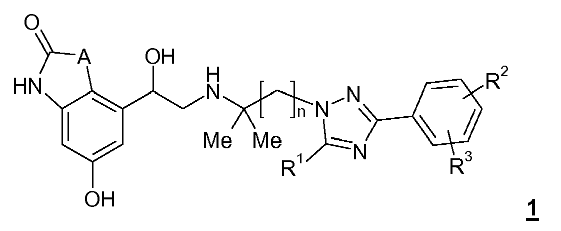 wherein
n denotes 1, 2 or 3, preferably 2;
A denotes a double-bonded group selected from among -CR⁴R⁵-O-, -CH=CH- or -CH₂-CH₂-, preferably -CR⁴R⁵-O-;
R¹ denotes -C₁₋₄-alkyl;
R² and R³, which may be identical or different, denote H, C₁₋₄-alkyl, C₂₋₄-alkenyl, C₂₋₄-alkynyl, C₃₋₆-cycloalkyl, C₁₋₄-haloalkyl, -O-C₁₋₄-haloalkyl, halogen, OH, CN, NO₂, -C₂₋₄-alkyl-OH, -O-C₁₋₄-alkyl, COOH or COO-C₁₋₄-alkyl, or R² and R³ together denote a double-bonded group selected from -O-CR⁴R⁵-O-, -O-CR⁴R⁵-NR⁶- or -CH=CH-CH=CH-;
R⁴ denotes H or C₁₋₄-alkyl;
R⁵ denotes H or C₁₋₄-alkyl;
R⁶ denotes H or C₁₋₄-alkyl,
optionally in the form of the individual enantiomers, mixtures of the individual enantiomers or racemates, optionally in the form of the pharmacologically acceptable acid addition salts thereof, as well as optionally in the form of the solvates and/or hydrates thereof.

2. Compounds of formula **1** according to claim 1,
wherein
A denotes a double-bonded group selected from among -CR⁴R⁵-O-, -CH=CH- or -CH₂-CH₂-, preferably -CR⁴R⁵-O- where
R⁴ denotes H, methyl, ethyl, preferably H or methyl, particularly preferably H;
R⁵ denotes H, methyl, ethyl, preferably H or methyl, particularly preferably H;
optionally in the form of the individual enantiomers, mixtures of the individual enantiomers or racemates, optionally in the form of the pharmacologically acceptable acid addition salts thereof, as well as optionally in the form of the solvates and/or hydrates thereof.

3. Compounds of formula **1** according to claim 1 or 2, wherein R¹ denotes methyl, ethyl or propyl, optionally in the form of the individual enantiomers, mixtures of the individual enantiomers or racemates, optionally in the form of the pharmacologically acceptable acid addition salts thereof, as well as optionally in the form of the solvates and/or hydrates thereof.

4. Compounds of formula **1** according to claim 1, 2 or 3, wherein
R² denotes H, methyl, ethyl, propyl, vinyl, allyl, propargyl, cyclopropyl, cyclopentyl, cyclohexyl, -CH₂Cl, -CHCl₂, -CCl₃, -CH₂F, -CHF₂, -CF₃, -CH₂-CH₂Cl, -CH₂-CHCl₂, -CH₂-CCl₃, -CH₂-CH₂F, -CH₂-CHF₂, -CH₂-CF₃, -CH₂-CH₂OH, fluorine, chlorine, bromine, OH, CN, NO₂, methoxy, ethoxy, propoxy, COOH, COO-methyl, COO-ethyl, COO-propyl or COO-butyl;
R³ denotes methyl, ethyl, propyl, vinyl, allyl, propargyl, cyclopropyl, cyclopentyl, cyclohexyl, -CH₂Cl, -CHCl₂, -CCl₃, -CH₂F, -CHF₂, -CF₃, -CH₂-CH₂Cl, -CH₂-CHCl₂, -CH₂-CCl₃, -CH₂-CH₂F, -CH₂-CHF₂, -CH₂-CF₃, -CH₂-CH₂OH, fluorine, chlorine, bromine, OH, CN, NO₂, methoxy, ethoxy, propoxy, COOH, COO-methyl, COO-ethyl, COO-propyl or COO-butyl, or R² and R³ together denote a double-bonded group selected from -O-CR⁴R⁵-O-, -O-CR⁴R⁵-NR⁶- or -CH=CH-CH=CH-;
R⁴ denotes H, methyl, ethyl, preferably H or methyl, particularly preferably H;
R⁵ denotes H, methyl, ethyl, preferably H or methyl, particularly preferably H;
R⁶ denotes H, methyl, ethyl, preferably H or methyl, particularly preferably H;
optionally in the form of the individual enantiomers, mixtures of the individual enantiomers or racemates, optionally in the form of the pharmacologically acceptable acid addition salts thereof, as well as optionally in the form of the solvates and/or hydrates thereof.

5. Compounds of formula **1** according to one of claims 1 to 4, **characterised in that** they are in the form of one of the acid addition salts thereof with pharmacologically acceptable acids which is selected from among the hydrochloride,
hydrobromide, hydriodide, hydrosulphate, hydrophosphate, hydromethanesulphonate, hydronitrate, hydromaleate, hydroacetate, hydrobenzoate, hydrocitrate, hydrofumarate, hydrotartrate, hydroxalate, hydrosuccinate, hydrobenzoate and hydro-p-toluenesulphonate.

6. Compounds of formula **1** according to one of claims 1 to 5, **characterised in that** they are in the form of the R-enantiomers of formula ***R*-1**

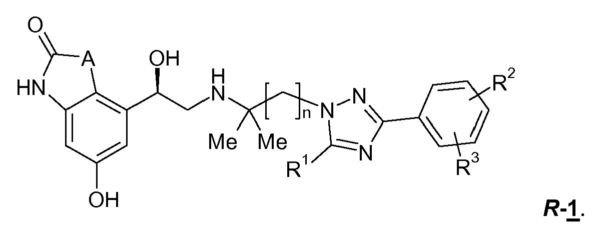

7. Compounds of formula **1** according to one of claims 1 to 6 as pharmaceutical compositions.

8. Use of the compounds of formula **1** according to one of claims 1 to 6 for preparing a pharmaceutical composition for the treatment of respiratory complaints.

9. Pharmaceutical formulation, **characterised in that** it contains a compound of formula **1** according to one of claims 1 to 6.

## Revendications

1. Composés de formule 1 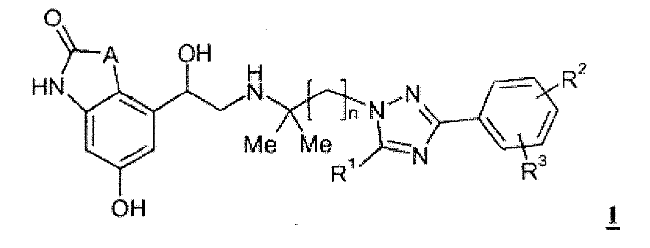 dans laquelle
n vaut 1, 2 ou 3, de préférence 2 ;
A est un groupe à double liaison choisi dans le groupe comprenant -CR⁴R⁵-O-, -CH=CH- ou -CH₂CH₂-, de préférence -CR⁴R⁵-O- ;
R¹ est un groupe alkyle en C₁ à C₄ ;
R² et R³, identiques ou différents sont H, un groupe allyle en C₁ à C₄, alcényle en C₂ à C₄, alcinyle en C₂ à C₄, cycloalkyle en C₃ à C₆, halogénoalkyle en C₁ à C₄, -O-halogénoalkyle en C₁ à C₄, halogène, OH, CN, NO₂, alkyle en C₂ à C₄-OH, -O-alkyle en C₁ à C₄, COOH ou COO-alkyle en C₁ à C₄, ou
R² et R³ conjointement sont un groupe à deux liaisons choisi parmi -O-CR⁴R⁵-O-, -O-CR⁴R⁵-NR⁶- ou -CH= CH-CH=CH- ;
R⁴ est H ou un groupe alkyle en C₁ à C₄ ;
R⁵ est H ou un groupe alkyle en C₁ à C₄ ;
R⁶ est H ou un groupe alkyle en C₁ à C₄, éventuellement sous la forme d'énantiomères individuels, de mélanges d'énantiomères individuels ou de racémates, éventuellement sous la forme de leurs sels d'addition acides pharmacologiquement acceptables, et éventuellement sous la forme de leurs solvates et/ou hydrates.

2. Composés de formule 1 selon la revendication 1, dans laquelle
A est un groupe à double liaison choisi dans le groupe comprenant -CR⁴R⁵-O-, -CH=CH- ou -CH₂CH₂-, de préférence -CR⁴R⁵-O- ; et
R⁴ est H, un groupe méthyle, éthyle, de préférence H ou un groupe méthyle, de manière particulièrement préférée, H ;
R⁵ est H, un groupe méthyle, éthyle, de préférence H ou un groupe méthyle, de manière particulièrement préférée, H, éventuellement sous la forme d'énantiomères individuels, de mélanges d'énantiomères individuels ou de racémates, éventuellement sous la forme de leurs sels d'addition acides pharmacologiquement acceptables, et éventuellement sous la forme de leurs solvates et/ou hydrates.

3. Composés de formule 1 selon la revendication 1 ou 2, dans laquelle R¹ est un groupe méthyle, éthyle ou propyle, éventuellement sous la forme des énantiomères individuels, de mélanges d'énantiomères individuels ou de racémates, éventuellement sous la forme de leurs sels d'addition acides pharmacologiquement acceptables et éventuellement sous la forme de leurs solvates et/ou hydrates.

4. Composés de formule 1 selon la revendication 1, 2 ou 3, dans laquelle
R² est H, un groupe méthyle, éthyle, propyle, vinyle, allyle, propargyle, cyclopropyle, cyclopentyle, cyclohexyle, -CH₂Cl, -CHCl₂, -CCl₃, -CH₂F, -CHF₂, -CF₃, -CH₂-CH₂Cl , -CH₂-CHCl₂, -CH₂-CCl₃, -CH₂-CH₂F, -CH₂-CHF₂, -CH₂-CF₃, -CH₂-CH₂OH, un atome de fluor, de chlore, de brome, OH, CN, NO₂, un groupe méthoxy, éthoxy, propoxy, COOH, COO-méthyle, COO-éthyle, COO-propyle ou COO-butyle ;
R³ est un groupe méthyle, éthyle, propyle, vinyle, allyle, propargyle, cyclopropyle, cyclopentyle, cyclohexyle, -CH₂Cl, -CHCl₂, -CCl₃, -CH₂F, -CHF₂, - CF₃, -CH₂-CH₂Cl, -CH₂-CHCl₂, -CH₂-CCl₃, -CH₂-CH₂F, - CH₂-CHF₂, -CH₂-CF₃, -CH₂-CH₂OH, un atome de fluor, de chlore, de brome, OH, CN, NO₂, un groupe méthoxy, éthoxy, propoxy, COOH, COO-méthyle, COO-éthyle, COO-propyle ou COO-butyle, ou
R² et R³ conjointement sont un groupe à deux liaisons choisi parmi -O-CR⁴R⁵-O-, -O-CR⁴R⁵-NR⁶- ou -CH=CH-CH=CH- ;
R⁴ est H, un groupe méthyle, éthyle, de préférence H ou méthyle, de manière particulièrement préférée H ;
R⁵ est H, un groupe méthyle, éthyle, de préférence H ou un groupe méthyle, de manière particulièrement préférée, H ;
R⁶ est H, un groupe méthyle, éthyle, de préférence H ou un groupe méthyle, de manière particulièrement préférée, H ;
éventuellement sous la forme d'énantiomères individuels, de mélanges d'énantiomères individuels ou de racémates, éventuellement sous la forme de leurs sels d'addition acides pharmacologiquement acceptables, et éventuellement sous la forme de leurs solvates et/ou hydrates.

5. Composés de formule 1 selon l'une des revendications 1 à 4, **caractérisé en ce qu'**ils se présentent sous la forme de l'un de leurs sels d'addition acides avec des acides pharmacologiquement acceptables qui sont choisis dans le groupe comprenant le chlorhydrate, le bromhydrate, l'iodhydrate, l'hydrosulfate, l'hydrophosphate, l'hydrométhanesulfonate, l'hydronitrate, l'hydromaléate, l'hydroacétate, l'hydrobenzoate, l'hydrocitrate, l'hydrofumarate, l'hydrotartrate, l'hydrooxalate, l'hydrosuccinate, l'hydrobenzoate et l'hydro-p-toluène-sulfonate.

6. Composés de formule 1 selon l'une des revendications 1 à 5, **caractérisés en ce qu'**ils se présentent sous la forme des énantiomères R de formule R-1

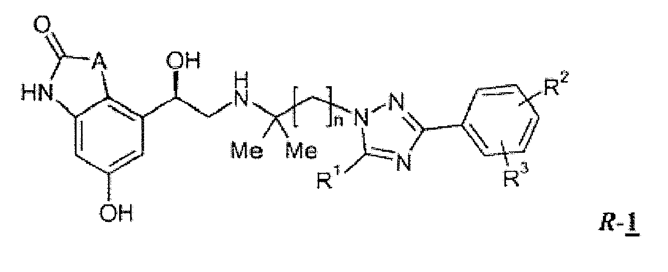

7. Composés de formule 1 selon l'une des revendications 1 à 6 comme médicament.

8. Utilisation des composés de formule 1 selon l'une des revendications 1 à 6, pour la production d'un médicament pour le traitement des affections respiratoires.

9. Formulation pharmaceutique **caractérisée par** une teneur en composé de formule 1 selon l'une des revendications 1 à 6.
